# EUROPEAN PATENT SPECIFICATION

(11) **EP 3 443 094 B1**
(45) Date of publication and mention of the grant of the patent: **19.10.2022**
(21) Application number: 17783110.4
(22) Date of filing: 13.04.2017
(51) Int. Cl.: C12N 15/113, C12N 15/11, C12N 15/09, A61K 31/7105, A61K 31/7115, A61K 31/712, A61P 25/28

(54) **METHODS FOR REDUCING C9ORF72 EXPRESSION**
VERFAHREN ZUR REDUZIERUNG DER C9ORF72-EXPRESSION
PROCÉDÉS DE RÉDUCTION DE L'EXPRESSION DE C9ORF72

(30) Priority: 13.04.2016 US 201662322146 P
(43) Date of publication of application: 20.02.2019
(73) Proprietor: Ionis Pharmaceuticals, Inc., Carlsbad, CA 92010 (US); LUDWIG INSTITUTE FOR CANCER RESEARCH, CH-8001 Zurich (CH)
(72) Inventor: BENNETT, C., Frank, Carlsbad, CA 92010 (US); RIGO, Frank, Carlsbad, CA 92010 (US); CLEVELAND, Don, W., La Jolla, CA 92093-0670 (US); JIANG, Jie, La Jolla, CA 92093-0670 (US); ZHU, Qiang, La Jolla, CA 92093-0670 (US); LAGIER-TOURENE, Clotilde, Charlestown, MA 02129 (US)
(74) Representative: Carpmaels & Ransford LLP
(86) International application number: PCT/US2017/027355
(87) International publication number: WO 2017/180835

(56) References cited:
- WO-A1-2015/057727
- WO-A1-2015/057738
- WO-A2-2015/054676
- WO-A2-2015/054676
- US-A1- 2015 259 679
- US-A1- 2015 267 197
- US-A1- 2016 304 871
- C. J. MAHONEY ET AL: "Frontotemporal dementia with the C9ORF72 hexanucleotide repeat expansion: clinical, neuroanatomical and neuropathological features", BRAIN, vol. 135, no. 3, 24 February 2012 (2012-02-24), pages 736-750, XP055429684, ISSN: 0006-8950, DOI: 10.1093/brain/awr361
- MAHONEY, CJ ET AL.: 'Frontotemporal dementia with the C90RF72 hexanucleotide repeat expansion: clinical, neuroanatomical and neuropathological features' BRAIN vol. 135, 2012, pages 736 - 750, XP055429684

## Description

### Field

Provided are methods for reducing the amount or activity of C9ORF72 RNA, and in certain instances of reducing the amount of C9ORF72 protein, in an animal. Such methods are useful to prevent or ameliorate at least one symptom of a neurodegenerative disease. Such symptoms include anxiety, reduced spatial learning, and memory loss. Such neurodegenerative diseases include amyotrophic lateral sclerosis (ALS), frontotemporal dementia (FTD), corticalbasal degeneration syndrome (CBD), atypical Parkinsonian syndrome, and olivopontocerellar degeneration (OPCD).

### Background

Amyotrophic lateral sclerosis (ALS) is a fatal neurodegenerative disease characterized clinically by progressive paralysis leading to death from respiratory failure, typically within two to three years of symptom onset (Rowland and Shneider, N. Engl. J. Med., 2001, 344, 1688-1700). ALS is the third most common neurodegenerative disease in the Western world (Hirtz et al., Neurology, 2007, 68, 326-337), and there are currently no effective therapies. Approximately 10% of cases are familial in nature, whereas the bulk of patients diagnosed with the disease are classified as sporadic as they appear to occur randomly throughout the population (Chio et al., Neurology, 2008, 70, 533-537). There is growing recognition, based on clinical, genetic, and epidemiological data, that ALS and frontotemporal dementia (FTD) represent an overlapping continuum of disease, characterized pathologically by the presence of TDP-43 positive inclusions throughout the central nervous system (Lillo and Hodges, J. Clin. Neurosci., 2009, 16, 1131-1135; Neumann et al., Science, 2006, 314, 130-133).

To date, a number of genes have been discovered as causative for classical familial ALS, for example, SOD1, TARDBP, FUS, OPTN, and VCP (Johnson et al., Neuron, 2010, 68, 857-864; Kwiatkowski et al., Science, 2009, 323, 1205-1208; Maruyama et al., Nature, 2010, 465, 223-226; Rosen et al., Nature, 1993, 362, 59-62; Sreedharan et al., Science, 2008, 319, 1668-1672; Vance et al., Brain, 2009, 129, 868-876). Recently, linkage analysis of kindreds involving multiple cases of ALS, FTD, and ALS-FTD had suggested that there was an important locus for the disease on the short arm of chromosome 9 (Boxer et al., J. Neurol. Neurosurg. Psychiatry, 2011, 82, 196-203; Morita et al., Neurology, 2006, 66, 839-844; Pearson et al. J. Nerol., 2011, 258, 647-655; Vance et al., Brain, 2006, 129, 868-876). The chromosome 9p21ALS-FTD locus in the last major autosomal-dominant gene whose mutation is causative of ALS. The ALS-FTD causing mutation is a large hexanucleotide (GGGGCC) repeat expansion in the first intron of the C9ORF72 gene (Renton et al., Neuron, 2011, 72, 257-268; DeJesus-Hernandez et al., Neuron, 2011, 72, 245-256). A founder haplotype, covering the C9ORF72 gene, is present in the majority of cases linked to this region (Renton et al., Neuron, 2011, 72, 257-268). This locus on chromosome 9p21 accounts for nearly half of familial ALS and nearly one-quarter of all ALS cases in a cohort of 405 Finnish patients (Laaksovirta et al, Lancet Neurol., 2010, 9, 978-985).

Currently there is a lack of acceptable options for treating neurodegenerative diseases such as amyotrophic lateral sclerosis (ALS), frontotemporal dementia (FTD), corticalbasal degeneration syndrome (CBD), atypical Parkinsonian syndrome, and olivopontocerellar degeneration (OPCD). It is therefore an object herein to provide methods for the treatment of such diseases.

WO 2015/054676, US 2015/259679 and US 2015/267197 describe compounds comprising modified oligonucleotides for reducing expression of C9ORF72 mRNA and/or protein in an animal that are useful for treating, preventing, or ameliorating neurodegenerative diseases. Mahoney *et al.,* 2012 provides a clinical, neuroanatomical and neuropathological analysis of cases with frontotemporal lobar degenerations (FTLD), confirming earlier reports that the C9ORF72 hexanucleotide repeat expansion is an important cause of FTLD.

### Summary of the Invention

The invention provides an oligomeric compound comprising a modified oligonucleotide for use in ameliorating or preventing at least one symptom of a neurodegenerative disease, wherein the modified oligonucleotide consists of 12 to 30 linked nucleosides, wherein the modified oligonucleotide has a nucleobase sequence that is at least 90% complementary to a C9ORF72 nucleic acid, wherein the modified oligonucleotide has a nucleobase sequence comprising at least 12, at least 13, at least 14, at least 15, at least 16, at least 17, or 18 contiguous nucleobases of SEQ ID NO: 6, and wherein the at least one symptom is any of anxiety, reduced spatial learning, and memory loss.

In certain embodiments, the neurodegenerative disease is amyotrophic lateral sclerosis (ALS), frontotemporal dementia (FTD), corticalbasal degeneration syndrome (CBD), atypical Parkinsonian syndrome, or olivopontocerellar degeneration (OPCD). In certain embodiments, amelioration of these symptoms results in reduced anxiety, improved spatial learning, or improved memory.

### Detailed Description of the Invention

It is to be understood that both the foregoing general description and the following detailed description are exemplary and explanatory only and are not restrictive. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature used in connection with, and the procedures and techniques of, analytical chemistry, synthetic organic chemistry, and medicinal and pharmaceutical chemistry described herein are those well known and commonly used in the art.

Unless otherwise indicated, the following terms have the following meanings:

### DEFINITIONS

"Administering" means providing a pharmaceutical agent to an animal. "Administered prior to the detection of the at least one symptom" is prophylactic administration and means providing the pharmaceutical agent to an animal before a symptom of a neurodegenerative disease is apparent through diagnosis. Such diagnosis may be accomplished by, for example, clinical evaluation and genetic testing.

"Animal" means a human or non-human animal.

"Antisense activity" means any detectable and/or measurable change attributable to the hybridization of an antisense compound to its target nucleic acid. In certain embodiments, antisense activity is a decrease in the amount or expression of a target nucleic acid or protein encoded by such target nucleic acid compared to target nucleic acid levels or target protein levels in the absence of the antisense compound.

"Ameliorate" or "amelioration" in reference to a treatment means improvement in at least one symptom relative to the same symptom in the absence of the treatment. In certain embodiments, amelioration is the reduction in the severity or frequency of a symptom or the delayed onset or slowing of progression in the severity or frequency of a symptom. The symptoms are anxiety, reduced spatial learning, and memory loss.

"Bicyclic sugar moiety" means a modified sugar moiety comprising two rings, wherein the second ring is formed via a bridge connecting two of the atoms in the first ring thereby forming a bicyclic structure. In certain embodiments, the first ring of the bicyclic sugar moiety is a furanosyl moiety. In certain embodiments, the bicyclic sugar moiety does not comprise a furanosyl moiety.

"Complementary" in reference to an oligonucleotide means that at least 70% of the nucleobases of the oligonucleotide or one or more regions thereof and the nucleobases of another nucleic acid or one or more regions thereof are capable of hydrogen bonding with one another when the nucleobase sequence of the oligonucleotide and the other nucleic acid are aligned in opposing directions. Complementary nucleobases means nucleobases that are capable of forming hydrogen bonds with one another. Complementary nucleobase pairs include, but unless otherwise specific are not limited to, adenine (A) and thymine (T), adenine (A) and uracil (U), cytosine (C) and guanine (G), 5-methyl cytosine (^{m}C) and guanine (G). Complementary oligonucleotides and/or nucleic acids need not have nucleobase complementarity at each nucleoside. Rather, some mismatches are tolerated. As used herein, "fully complementary" or "100% complementary" in reference to oligonucleotides means that oligonucleotides are complementary to another oligonucleotide or nucleic acid at each nucleoside of the oligonucleotide.

"Conjugate group" means a group of atoms that is directly or indirectly attached to an oligonucleotide. Conjugate groups include a conjugate moiety and a conjugate linker that attaches the conjugate moiety to the oligonucleotide.

"Contiguous" in the context of an oligonucleotide refers to nucleosides, nucleobases, sugar moieties, or internucleoside linkages that are immediately adjacent to each other. For example, "contiguous nucleobases" means nucleobases that are immediately adjacent to each other in a sequence.

"Gapmer" means a modified oligonucleotide comprising an internal region having a plurality of nucleosides that support RNase H cleavage positioned between external regions having one or more nucleosides, wherein the nucleosides comprising the internal region are chemically distinct from the nucleoside or nucleosides comprising the external regions. The internal region may be referred to as the "gap" and the external regions may be referred to as the "wings."

"Internucleoside linkage" is the covalent linkage between adjacent nucleosides in an oligonucleotide. As used herein "modified internucleoside linkage" means any internucleoside linkage other than a phosphodiester internucleoside linkage. "Phosphorothioate linkage" means a modified internucleoside linkage in which one of the non-bridging oxygen atoms of a phosphodiester internucleoside linkage is replaced with a sulfur atom.

"MOE" means methoxyethyl. "2'-MOE" means a -OCH₂CH₂OCH₃ group at the 2' position of a furanosyl ring.

"Neurodegenerative disease" means a condition marked by progressive loss of structure or function of neurons, including death of neurons. In certain emodiments, neurodegenerative disease is any of amyotrophic lateral sclerosis (ALS), frontotemporal dementia (FTD), corticalbasal degeneration syndrome (CBD), atypical Parkinsonian syndrome, and olivopontocerebellar degeneration (OPCD).

"Nucleobase" means an unmodified nucleobase or a modified nucleobase. As used herein "an "unmodified nucleobase" is adenine (A), thymine (T), cytosine (C), uracil (U), and guanine (G). As used herein, a "modified nucleobase" is a group of atoms other than unmodified A, T, C, U, or G capable of pairing with at least one unmodified nucleobase. A "5-methylcytosine" is a modified nucleobase. A universal base is a modified nucleobase that can pair with any one of the five unmodified nucleobases. As used herein, "nucleobase sequence" means the order of contiguous nucleobases (*i.e.* no additional nucleobases are present between those that are contiguous) in a nucleic acid or oligonucleotide independent of any sugar or internucleoside linkage modification.

"Nucleoside" means a compound comprising a nucleobase and a sugar moiety. The nucleobase and sugar moiety are each, independently, unmodified or modified. As used herein, "modified nucleoside" means a nucleoside comprising a modified nucleobase and/or a modified sugar moiety. Modified nucleosides include abasic nucleosides, which lack a nucleobase. "Linked nucleosides" are nucleosides that are connected in a continuous sequence (*i.e*. no additional nucleosides are present between those that are linked).

"Oligomeric compound" means a compound comprising an oligonucleotide and optionally one or more additional features, such as a conjugate group or terminal group.

"Oligomeric compound" means an oligonucleotide and optionally one or more additional features, such as a conjugate group or terminal group. An oligomeric compound may be paired with a second oligomeric compound or may be unpaired. A "singled-stranded oligomeric compound" is an unpaired oligomeric compound. A "duplexed oligomeric compound" is an oligomeric comopund paired with a second oligomeric compound; this is an "oligomeric duplex."

"Oligonucleotide" means a strand of linked nucleosides connected via internucleoside linkages, wherein each nucleoside and internucleoside linkage may be modified or unmodified. Unless otherwise indicated, oligonucleotides consist of 8-50 linked nucleosides. As used herein, "modified oligonucleotide" means an oligonucleotide, wherein at least one nucleoside or internucleoside linkage is modified. As used herein, "unmodified oligonucleotide" means an oligonucleotide that does not comprise any nucleoside modifications or internucleoside modifications.

"Reducing or inhibiting the expression or amount" refers to a reduction or blockade of the expression or amount relative to the expression or amount in an untreated or control sample and does not necessarily indicate a total elimination of expression or amount.

"Salts" mean physiologically and pharmaceutically acceptable salts of oligomeric compounds, i.e., salts that retain the desired biological activity of the parent oligomeric compound and do not impart undesired toxicological effects thereto.

"Standard cell assay" means the assay described in Example 5 and reasonable variations thereof.

"Standard in vivo experiment" means the procedure described in Example 7 and reasonable variations thereof.

"Sugar moiety" means an unmodified sugar moiety or a modified sugar moiety. As used herein, "unmodified sugar moiety" means a 2'-OH(H) furanosyl moiety, as found in RNA (an "unmodified RNA sugar moiety"), or a 2'-H(H) moiety, as found in DNA (an "unmodified DNA sugar moiety"). Unmodified sugar moieties have one hydrogen at each of the 1', 3', and 4' positions, an oxygen at the 3' position, and two hydrogens at the 5' position. As used herein, "modified sugar moiety" means a modified furanosyl sugar moiety or a sugar surrogate. As used herein, modified furanosyl sugar moiety means a furanosyl sugar comprising a non-hydrogen substituent in place of at least one hydrogen of an unmodified sugar moiety. Modified furanosyl sugar moieties include bicyclic sugars and non-bicyclic sugars. As used herein, "sugar surrogate" means a modified sugar moiety having other than a furanosyl moiety that can link a nucleobase to another group, such as an internucleoside linkage, conjugate group, or terminal group in an oligonucleotide. Modified nucleosides comprising sugar surrogates can be incorporated into one or more positions within an oligonucleotide and such oligonucleotides are capable of hybridizing to complementary oligomeric compounds or nucleic acids.

"Target nucleic acid" means a nucleic acid to which an oligomeric compound is designed to hybridize.

"Therapeutically effective amount" means an amount of a pharmaceutical agent that provides a therapeutic benefit to an animal. For example, a therapeutically effective amount improves a symptom of a disease.

"Total C9ORF72 protein" means all C9ORF72 protein variants.

"Total C9ORF72 RNA" means all C9ORF72 RNA variants. "Pathogenic C9ORF72 RNA" means variants containing an expanded hexanucleotide repeat.

### I. Certain Oligonucleotides

Described herein are oligonucleotides, which consist of linked nucleosides. Oligonucleotides may be unmodified oligonucleotides (RNA or DNA) or may be modified oligonucleotides. Modified oligonucleotides comprise at least one modification relative to unmodified RNA or DNA. That is, modified oligonucleotides comprise at least one modified nucleoside (comprising a modified sugar moiety and/or a modified nucleobase) and/or at least one modified internucleoside linkage.

### A. Certain Modified Nucleosides

Modified nucleosides comprise a modified sugar moiety or a modified nucleobase or both a modifed sugar moiety and a modified nucleobase.

### 1. Certain Sugar Moieties

In certain embodiments, modified sugar moieties are non-bicyclic modified sugar moieties. In certain embodiments, modified sugar moieties are bicyclic or tricyclic sugar moieties. In certain embodiments, modified sugar moieties are sugar surrogates. Such sugar surrogates may comprise one or more substitutions corresponding to those of other types of modified sugar moieties.

In certain embodiments, modified sugar moieties are non-bicyclic modified sugar moieties comprising a furanosyl ring with one or more acyclic substituent, including but not limited to substituents at the 2', 4', and/or 5' positions. In certain embodiments one or more acyclic substituent of non-bicyclic modified sugar moieties is branched. Examples of 2'-substituent groups suitable for non-bicyclic modified sugar moieties include but are not limited to: 2'-F, 2'-OCH₃ ("OMe" or "O-methyl"), and 2'-O(CH₂)₂OCH₃ ("MOE"). In certain embodiments, 2'-substituent groups are selected from among: halo, allyl, amino, azido, SH, CN, OCN, CF₃, OCF₃, O-C₁-C₁₀ alkoxy, O-C₁-C₁₀ substituted alkoxy, O-C₁-C₁₀ alkyl, O-C₁-C₁₀ substituted alkyl, S-alkyl, N(Rₘ)-alkyl, O-alkenyl, S-alkenyl, N(Rₘ)-alkenyl, O-alkynyl, S-alkynyl, N(Rₘ)-alkynyl, O-alkylenyl-O-alkyl, alkynyl, alkaryl, aralkyl, O-alkaryl, O-aralkyl, O(CH₂)₂SCH₃, O(CH₂)₂ON(Rₘ)(Rₙ) or OCH₂C(=O)-N(Rₘ)(Rₙ), where each Rₘ and Rₙ is, independently, H, an amino protecting group, or substituted or unsubstituted C₁-C₁₀ alkyl, and the 2'-substituent groups described in Cook et al., U.S. 6,531,584; Cook et al., U.S. 5,859,221; and Cook et al., U.S. 6,005,087. Certain embodiments of these 2'-substituent groups can be further substituted with one or more substituent groups independently selected from among: hydroxyl, amino, alkoxy, carboxy, benzyl, phenyl, nitro (NO₂), thiol, thioalkoxy, thioalkyl, halogen, alkyl, aryl, alkenyl and alkynyl. Examples of 4'-substituent groups suitable for non-bicyclic modified sugar moieties include but are not limited to alkoxy (e.g., methoxy), alkyl, and those described in Manoharan et al., WO 2015/106128. Examples of 5'-substituent groups suitable for non-bicyclic modified sugar moieties include but are not limited to: 5'-methyl (R or S), 5'-vinyl, and 5'-methoxy. In certain embodiments, non-bicyclic modified sugar moieties comprise more than one non-bridging sugar substituent, for example, 2'-F-5'-methyl sugar moieties and the modified sugar moieties and modified nucleosides described in Migawa et al., WO 2008/101157 and Rajeev et al., US2013/0203836.).

In certain embodiments, a 2'-substituted nucleoside or 2'- non-bicyclic modified nucleoside comprises a sugar moiety comprising a non-bridging 2'-substituent group selected from: F, NH₂, N₃, OCF₃, OCH₃, O(CH₂)₃NH₂, CH₂CH=CH₂, OCH₂CH=CH₂, OCH₂CH₂OCH₃, O(CH₂)₂SCH₃,O(CH₂)₂ON(Rₘ)(Rₙ), O(CH₂)₂O(CH₂)₂N(CH₃)₂, and N-substituted acetamide (OCH₂C(=O)-N(Rₘ)(Rₙ)), where each Rₘ and Rₙ is, independently, H, an amino protecting group, or substituted or unsubstituted C₁-C₁₀ alkyl.

In certain embodiments, a 2'-substituted nucleoside or 2'- non-bicyclic modified nucleoside comprises a sugar moiety comprising a non-bridging 2'-substituent group selected from: F, OCF₃, OCH₃, OCH₂CH₂OCH₃, O(CH₂)₂SCH₃,O(CH₂)₂ON(CH₃)₂, O(CH₂)₂O(CH₂)₂N(CH₃)₂, and OCH₂C(=O)-N(H)CH₃ ("NMA").

In certain embodiments, a 2'-substituted nucleoside or 2'- non-bicyclic modified nucleoside comprises a sugar moiety comprising a non-bridging 2'-substituent group selected from: F, OCH₃, and OCH₂CH₂OCH₃.

Nucleosides comprising modified sugar moieties, such as non-bicyclic modified sugar moieties, may be referred to by the position(s) of the substitution(s) on the sugar moiety of the nucleoside. For example, nucleosides comprising 2'-substituted sugar moieties are referred to as 2'-substituted nucleosides.

Certain modifed sugar moieties comprise a bridging sugar substituent that forms a second ring resulting in a bicyclic sugar moiety. In certain such embodiments, the bicyclic sugar moiety comprises a bridge between the 4' and the 2' furanose ring atoms. Examples of such 4' to 2' bridging sugar substituents include but are not limited to: 4'-CH₂-2", 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt" when in the S configuration), 4'-CH₂-O-CH₂-2', 4'-CH₂-N(R)-2', 4'-CH(CH₂OCH₃)-O-2' ("constrained MOE" or "cMOE") and analogs thereof (*see, e.g.,* Seth et al., U.S. 7,399,845, Bhat et al., U.S. 7,569,686, Swayze et al., U.S. 7,741,457, and Swayze et al., U.S. 8,022,193), 4'-C(CH₃)(CH₃)-O-2' and analogs thereof (*see, e.g.,* Seth et al., U.S. 8,278,283), 4'-CH₂-N(OCH₃)-2' and analogs thereof (*see, e.g.,* Prakash et al., U.S. 8,278,425), 4'-CH₂-ON(CH₃)-2' (*see, e.g.,* Allerson et al., U.S. 7,696,345 and Allerson et al., U.S. 8,124,745), 4'-CH₂-C(H)(CH₃)-2' (*see, e.g.,* Zhou, et al., J. Org. Chem.,2009, 74, 118-134), 4'-CH₂-C(=CH₂)-2' and analogs thereof (*see e.g.,* Seth et al., U.S. 8,278,426), 4'-C(RₐR_{b})-N(R)-O-2', 4'-C(RₐR_{b})-O-N(R)-2' 4'-CH₂-O-N(R)-2', and 4'-CH₂-N(R)-O-2', wherein each R, Rₐ, and R_{b} is, independently, H, a protecting group, or C₁-C₁₂ alkyl (*see, e.g.* Imanishi et al., U.S. 7,427,672).

In certain embodiments, such 4' to 2' bridges independently comprise from 1 to 4 linked groups independently selected from: -[C(Rₐ)(R_{b})]ₙ-, -[C(Rₐ)(R_{b})]ₙ-O-, -C(Rₐ)=C(R_{b})-, -C(Rₐ)=N-, -C(=NRₐ)-, - C(=O)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Additional bicyclic sugar moieties are known in the art, see, for example: Freier et al., Nucleic Acids Research, 1997, 25(22), 4429-4443, Albaek et al., J. Org. Chem., 2006, 71, 7731-7740, Singh et al., Chem. Commun., 1998, 4, 455-456; Koshkin et al., Tetrahedron, 1998, 54, 3607-3630; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 20017, 129, 8362-8379;Wengel et a., U.S. 7,053,207; Imanishi et al., U.S. 6,268,490; Imanishi et al. U.S. 6,770,748; Imanishi et al., U.S. RE44,779; Wengel et al., U.S. 6,794,499; Wengel et al., U.S. 6,670,461; Wengel et al., U.S. 7,034,133; Wengel et al., U.S. 8,080,644; Wengel et al., U.S. 8,034,909; Wengel et al., U.S. 8,153,365; Wengel et al., U.S. 7,572,582; and Ramasamy et al., U.S. 6,525,191;; Torsten et al., WO 2004/106356;Wengel et al., WO 1999/014226; Seth et al., WO 2007/134181; Seth et al., U.S. 7,547,684; Seth et al., U.S. 7,666,854; Seth et al., U.S. 8,088,746; Seth et al., U.S. 7,750,131; Seth et al., U.S. 8,030,467; Seth et al., U.S. 8,268,980; Seth et al., U.S. 8,546,556; Seth et al., U.S. 8,530,640; Migawa et al., U.S. 9,012,421; Seth et al., U.S. 8,501,805; and U.S. Patent Publication Nos. Allerson et al., US2008/0039618 and Migawa et al., US2015/0191727.

In certain embodiments, bicyclic sugar moieties and nucleosides incorporating such bicyclic sugar moieties are further defined by isomeric configuration. For example, an LNA nucleoside (described herein) may be in the α-L configuration or in the β-D configuration. 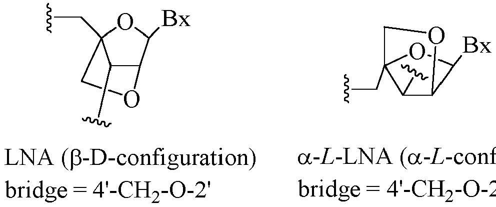 α-L-methyleneoxy (4'-CH₂-O-2') or α-L-LNA bicyclic nucleosides have been incorporated into oligonucleotides that showed antisense activity (Frieden et al., Nucleic Acids Research, 2003, 21, 6365-6372). Herein, general descriptions of bicyclic nucleosides include both isomeric configurations. When the positions of specific bicyclic nucleosides (*e.g*., LNA or cEt) are identified in exemplified embodiments herein, they are in the β-D configuration, unless otherwise specified.

In certain embodiments, modified sugar moieties comprise one or more non-bridging sugar substituent and one or more bridging sugar substituent (e.g., 5'-substituted and 4'-2' bridged sugars).

In certain embodiments, modified sugar moieties are sugar surrogates. In certain such embodiments, the oxygen atom of the sugar moiety is replaced, *e.g*., with a sulfur, carbon or nitrogen atom. In certain such embodiments, such modified sugar moieties also comprise bridging and/or non-bridging substituents as described herein. For example, certain sugar surrogates comprise a 4'-sulfur atom and a substitution at the 2'-position (*see, e.g.,* Bhat et al., U.S. 7,875,733 and Bhat et al., U.S. 7,939,677) and/or the 5' position.

In certain embodiments, sugar surrogates comprise rings having other than 5 atoms. For example, in certain embodiments, a sugar surrogate comprises a six-membered tetrahydropyran ("THP"). Such tetrahydropyrans may be further modified or substituted. Nucleosides comprising such modified tetrahydropyrans include but are not limited to hexitol nucleic acid ("HNA"), anitol nucleic acid ("ANA"), manitol nucleic acid ("MNA") (*see, e.g.,* Leumann, CJ. Bioorg. & Med. Chem. 2002, 10, 841-854), fluoro HNA: 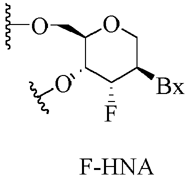 ("F-HNA", *see e.g*.Swayze et al., U.S. 8,088,904; Swayze et al., U.S. 8,440,803; Swayze et al., U.S. 8,796,437; and Swayze et al., U.S. 9,005,906; F-HNA can also be referred to as a F-THP or 3'-fluoro tetrahydropyran), and nucleosides comprising additional modified THP compounds having the formula: 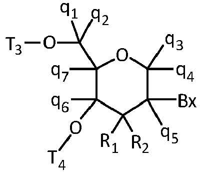 wherein, independently, for each of said modified THP nucleoside:
Bx is a nucleobase moiety;
T₃ and T₄ are each, independently, an internucleoside linking group linking the modified THP nucleoside to the remainder of an oligonucleotide or one of T₃ and T₄ is an internucleoside linking group linking the modified THP nucleoside to the remainder of an oligonucleotide and the other of T₃ and T₄ is H, a hydroxyl protecting group, a linked conjugate group, or a 5' or 3'-terminal group;
   q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from among: hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J₁, J₂, and J₃ is, independently, H or C₁-C₆ alkyl.

In certain embodiments, modified THP nucleosides are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, modified THP nucleosides are provided wherein one of R₁ and R₂ is F. In certain embodiments, R₁ is F and R₂ is H, in certain embodiments, R₁ is methoxy and R₂ is H, and in certain embodiments, R₁ is methoxyethoxy and R₂ is H.

In certain embodiments, sugar surrogates comprise rings having more than 5 atoms and more than one heteroatom. For example, nucleosides comprising morpholino sugar moieties and their use in oligonucleotides have been reported *(see, e.g.,* Braasch et al., Biochemistry, 2002, 41, 4503-4510 and Summerton et al., U.S. 5,698,685; Summerton et al., U.S. 5,166,315; Summerton et al., U.S. 5,185,444; and Summerton et al., U.S. 5,034,506). As used here, the term "morpholino" means a sugar surrogate having the following structure: 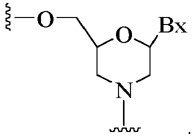 In certain embodiments, morpholinos may be modified, for example by adding or altering various substituent groups from the above morpholino structure. Such sugar surrogates are refered to herein as "modifed morpholinos."

In certain embodiments, sugar surrogates comprise acyclic moieites. Examples of nucleosides and oligonucleotides comprising such acyclic sugar surrogates include but are not limited to: peptide nucleic acid ("PNA"), acyclic butyl nucleic acid (*see, e.g.,* Kumar et al., Org. Biomol. Chem., 2013, 11, 5853-5865), and nucleosides and oligonucleotides described in Manoharan et al., WO2011/133876.

Many other bicyclic and tricyclic sugar and sugar surrogate ring systems are known in the art that can be used in modified nucleosides).

### 2. Certain Modified Nucleobases

In certain embodiments, modified oligonucleotides comprise one or more nucleoside comprising an unmodified nucleobase. In certain embodiments, modified oligonucleotides comprise one or more nucleoside comprising a modified nucleobase. In certain embodiments, modified oligonucleotides comprise one or more nucleoside that does not comprise a nucleobase, referred to as an abasic nucleoside.

In certain embodiments, modified nucleobases are selected from: 5-substituted pyrimidines, 6-azapyrimidines, alkyl or alkynyl substituted pyrimidines, alkyl substituted purines, and N-2, N-6 and O-6 substituted purines. In certain embodiments, modified nucleobases are selected from: 2-aminopropyladenine, 5-hydroxymethyl cytosine, xanthine, hypoxanthine, 2-aminoadenine, 6-N-methylguanine, 6-N-methyladenine, 2-propyladenine , 2-thiouracil, 2-thiothymine and 2-thiocytosine, 5-propynyl (-C≡C-CH₃) uracil, 5-propynylcytosine, 6-azouracil, 6-azocytosine, 6-azothymine, 5-ribosyluracil (pseudouracil), 4-thiouracil, 8-halo, 8-amino, 8-thiol, 8-thioalkyl, 8-hydroxyl, 8-aza and other 8-substituted purines, 5-halo, particularly 5-bromo, 5-trifluoromethyl, 5-halouracil, and 5-halocytosine, 7-methylguanine, 7-methyladenine, 2-F-adenine, 2-aminoadenine, 7-deazaguanine, 7-deazaadenine, 3-deazaguanine, 3-deazaadenine, 6-N-benzoyladenine, 2-N-isobutyrylguanine, 4-N-benzoylcytosine, 4-N-benzoyluracil, 5-methyl 4-N-benzoylcytosine, 5-methyl 4-N-benzoyluracil, universal bases, hydrophobic bases, promiscuous bases, size-expanded bases, and fluorinated bases. Further modified nucleobases include tricyclic pyrimidines, such as 1,3 -diazaphenoxazine-2-one, 1,3 -diazaphenothiazine-2-one and 9-(2-aminoethoxy)-1,3 -diazaphenoxazine-2-one (G-clamp). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in Merigan et al., U.S. 3,687,808, those disclosed in The Concise Encyclopedia Of Polymer Science And Engineering, Kroschwitz, J.I., Ed., John Wiley & Sons, 1990, 858-859; Englisch et al., Angewandte Chemie, International Edition, 1991, 30, 613; Sanghvi, Y.S., Chapter 15, Antisense Research and Applications, Crooke, S.T. and Lebleu, B., Eds., CRC Press, 1993, 273-288; and those disclosed in Chapters 6 and 15, Antisense Drug Technology, Crooke S.T., Ed., CRC Press, 2008, 163-166 and 442-443.

Publications that teach the preparation of certain of the above noted modified nucleobases as well as other modified nucleobases include without limitation, Manohara et al., US2003/0158403; Manoharan et al., US2003/0175906; Dinh et al., U.S. 4,845,205; Spielvogel et al., U.S. 5,130,302; Rogers et al., U.S. 5,134,066; Bischofberger et al., U.S. 5,175,273; Urdea et al., U.S. 5,367,066; Benner et al., U.S. 5,432,272; Matteucci et al., U.S. 5,434,257; Gmeiner et al., U.S. 5,457,187; Cook et al., U.S. 5,459,255; Froehler et al., U.S. 5,484,908; Matteucci et al., U.S. 5,502,177; Hawkins et al., U.S. 5,525,711; Haralambidis et al., U.S. 5,552,540; Cook et al., U.S. 5,587,469; Froehler et al., U.S. 5,594,121; Switzer et al., U.S. 5,596,091; Cook et al., U.S. 5,614,617; Froehler et al., U.S. 5,645,985; Cook et al., U.S. 5,681,941; Cook et al., U.S. 5,811,534; Cook et al., U.S. 5,750,692; Cook et al., U.S. 5,948,903; Cook et al., U.S. 5,587,470; Cook et al., U.S. 5,457,191; Matteucci et al., U.S. 5,763,588; Froehler et al., U.S. 5,830,653; Cook et al., U.S. 5,808,027; Cook et al., 6,166,199; and Matteucci et al., U.S. 6,005,096.

### 3. Certain Modified Internucleoside Linkages

In certain embodiments, nucleosides of modified oligonucleotides may be linked together using any internucleoside linkage. The two main classes of internucleoside linking groups are defined by the presence or absence of a phosphorus atom. Representative phosphorus-containing internucleoside linkages include but are not limited to phosphates, which contain a phosphodiester bond ("P=O") (also referred to as unmodified or naturally occurring linkages), phosphotriesters, methylphosphonates, phosphoramidates, and phosphorothioates ("P=S"), and phosphorodithioates ("HS-P=S"). Representative non-phosphorus containing internucleoside linking groups include but are not limited to methylenemethylimino (-CH₂-N(CH₃)-O-CH₂-), thiodiester, thionocarbamate (-O-C(=O)(NH)-S-); siloxane (-O-SiH₂-O-); and N,N'-dimethylhydrazine (-CH₂-N(CH₃)-N(CH₃)-). Modified internucleoside linkages, compared to naturally occurring phosphate linkages, can be used to alter, typically increase, nuclease resistance of the oligonucleotide. In certain embodiments, internucleoside linkages having a chiral atom can be prepared as a racemic mixture, or as separate enantiomers. Representative chiral internucleoside linkages include but are not limited to alkylphosphonates and phosphorothioates. Methods of preparation of phosphorous-containing and non-phosphorous-containing internucleoside linkages are well known to those skilled in the art.

Neutral internucleoside linkages include, without limitation, phosphotriesters, methylphosphonates, MMI (3'-CH_{z}-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), methoxypropyl, and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### B. Certain Motifs

In certain embodiments, modified oligonucleotides comprise one or more modified nucleosides comprising a modified sugar moiety. In certain embodiments, modified oligonucleotides comprise one or more modified nucleosides comprising a modified nucleobase. In certain embodiments, modified oligonucleotides comprise one or more modified internucleoside linkage. In such embodiments, the modified, unmodified, and differently modified sugar moieties, nucleobases, and/or internucleoside linkages of a modified oligonucleotide define a pattern or motif. In certain embodiments, the patterns of sugar moieties, nucleobases, and internucleoside linkages are each independent of one another. Thus, a modified oligonucleotide may be described by its sugar motif, nucleobase motif and/or internucleoside linkage motif (as used herein, nucleobase motif describes the modifications to the nucleobases independent of the sequence of nucleobases).

### 1. Certain Sugar Motifs

In certain embodiments, oligonucleotides comprise one or more type of modified sugar and/or unmodified sugar moiety arranged along the oligonucleotide or region thereof in a defined pattern or sugar motif. In certain instances, such sugar motifs include but are not limited to any of the sugar modifications discussed herein.

In certain embodiments, modified oligonucleotides comprise or consist of a region having a gapmer motif, which comprises two external regions or "wings" and a central or internal region or "gap." The three regions of a gapmer motif (the 5'-wing, the gap, and the 3'-wing) form a contiguous sequence of nucleosides wherein at least some of the sugar moieties of the nucleosides of each of the wings differ from at least some of the sugar moieties of the nucleosides of the gap. Specifically, at least the sugar moieties of the nucleosides of each wing that are closest to the gap (the 3'-most nucleoside of the 5'-wing and the 5'-most nucleoside of the 3'-wing) differ from the sugar moiety of the neighboring gap nucleosides, thus defining the boundary between the wings and the gap (i.e., the wing/gap junction). In certain embodiments, the sugar moieties within the gap are the same as one another. In certain embodiments, the gap includes one or more nucleoside having a sugar moiety that differs from the sugar moiety of one or more other nucleosides of the gap. In certain embodiments, the sugar motifs of the two wings are the same as one another (symmetric gapmer). In certain embodiments, the sugar motif of the 5'-wing differs from the sugar motif of the 3'-wing (asymmetric gapmer).

In certain embodiments, the wings of a gapmer comprise 1-5 nucleosides. In certain embodiments, each nucleoside of each wing of a gapmer is a modified nucleoside.

In certain embodiments, the gap of a gapmer comprises 7-12 nucleosides. In certain embodiments, each nucleoside of the gap of a gapmer is an unmodified 2'-deoxy nucleoside.

In certain embodiments, the gapmer is a deoxy gapmer. In embodiments, the nucleosides on the gap side of each wing/gap junction are unmodified 2'-deoxy nucleosides and the nucleosides on the wing sides of each wing/gap junction are modified nucleosides. In certain embodiments, each nucleoside of the gap is an unmodified 2'-deoxy nucleoside. In certain embodiments, each nucleoside of each wing of a gapmer is a modified nucleoside.

In certain embodiments, modified oligonucleotides comprise or consist of a region having a fully modified sugar motif. In such embodiments, each nucleoside of the fully modified region of the modified oligonucleotide comprises a modified sugar moiety. In certain embodiments, each nucleoside of the entire modified oligonucleotide comprises a modified sugar moiety. In certain embodiments, modified oligonucleotides comprise or consist of a region having a fully modified sugar motif, wherein each nucleoside within the fully modified region comprises the same modified sugar moiety, referred to herein as a uniformly modified sugar motif. In certain embodiments, a fully modified oligonucleotide is a uniformly modified oligonucleotide. In certain embodiments, each nucleoside of a uniformly modified comprises the same 2'-modification.

### 2. Certain Nucleobase Motifs

In certain embodiments, oligonucleotides comprise modified and/or unmodified nucleobases arranged along the oligonucleotide or region thereof in a defined pattern or motif. In certain embodiments, each nucleobase is modified. In certain embodiments, none of the nucleobases are modified. In certain embodiments, each purine or each pyrimidine is modified. In certain embodiments, each adenine is modified. In certain embodiments, each guanine is modified. In certain embodiments, each thymine is modified. In certain embodiments, each uracil is modified. In certain embodiments, each cytosine is modified. In certain embodiments, some or all of the cytosine nucleobases in a modified oligonucleotide are 5-methylcytosines.

In certain embodiments, modified oligonucleotides comprise a block of modified nucleobases. In certain such embodiments, the block is at the 3'-end of the oligonucleotide. In certain embodiments the block is within 3 nucleosides of the 3'-end of the oligonucleotide. In certain embodiments, the block is at the 5'-end of the oligonucleotide. In certain embodiments the block is within 3 nucleosides of the 5'-end of the oligonucleotide.

In certain embodiments, oligonucleotides having a gapmer motif comprise a nucleoside comprising a modified nucleobase. In certain such embodiments, one nucleoside comprising a modified nucleobase is in the central gap of an oligonucleotide having a gapmer motif. In certain such embodiments, the sugar moiety of said nucleoside is a 2'-deoxyribosyl moiety. In certain embodiments, the modified nucleobase is selected from: a 2-thiopyrimidine and a 5-propynepyrimidine.

### 3. Certain Internucleoside Linkage Motifs

In certain embodiments, oligonucleotides comprise modified and/or unmodified internucleoside linkages arranged along the oligonucleotide or region thereof in a defined pattern or motif. In certain embodiments, each internucleoside linking group is a phosphodiester internucleoside linkage (P=O). In certain embodiments, each internucleoside linking group of a modified oligonucleotide is a phosphorothioate internucleoside linkage (P=S). In certain embodiments, each internucleoside linkage of a modified oligonucleotide is independently selected from a phosphorothioate internucleoside linkage and phosphodiester internucleoside linkage. In certain embodiments, the sugar motif of a modified oligonucleotide is a gapmer and the internucleoside linkages within the gap are all modified. In certain such embodiments, some or all of the internucleoside linkages in the wings are unmodified phosphate linkages. In certain embodiments, the terminal internucleoside linkages are modified.

### C. Certain Lengths

It is possible to increase or decrease the length of an oligonuclotide without eliminating activity. For example, in Woolf et al. (Proc. Natl. Acad. Sci. USA 89:7305-7309, 1992), a series of oligonucleotides 13-25 nucleobases in length were tested for their ability to induce cleavage of a target RNA in an oocyte injection model. Oligonucleotides 25 nucleobases in length with 8 or 11 mismatch bases near the ends of the oligonucleotides were able to direct specific cleavage of the target mRNA, albeit to a lesser extent than the oligonucleotides that contained no mismatches. Similarly, target specific cleavage was achieved using 13 nucleobase oligonucleotides, including those with 1 or 3 mismatches.

Oligonucleotides (including modified oligonucleotides) can have any of a variety of ranges of lengths. Oligonucleotides may consist of X to Y linked nucleosides, where X represents the fewest number of nucleosides in the range and Y represents the largest number nucleosides in the range. X and Y can each be independently selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50; provided that X≤Y. For example, oligonucleotides may consist of 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 29, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 29 to 30 linked nucleosides

### D. Certain Modified Oligonucleotides

In certain embodiments, the above modifications (sugar, nucleobase, internucleoside linkage) are incorporated into a modified oligonucleotide. In certain embodiments, modified oligonucleotides are characterized by their modification motifs and overall lengths. In certain embodiments, such parameters are each independent of one another. Thus, unless otherwise indicated, each internucleoside linkage of an oligonucleotide having a gapmer sugar motif may be modified or unmodified and may or may not follow the gapmer modification pattern of the sugar modifications. For example, the internucleoside linkages within the wing regions of a sugar gapmer may be the same or different from one another and may be the same or different from the internucleoside linkages of the gap region of the sugar motif. Likewise, such sugar gapmer oligonucleotides may comprise one or more modified nucleobase independent of the gapmer pattern of the sugar modifications. Unless otherwise indicated, all modifications are independent of nucleobase sequence.

### E. Nucleobase Sequence

Oligonucleotides (unmodified or modified oligonucleotides) can be further described by their nucleobase sequence. Oligonucleotides may have a nucleobase sequence that is complementary to a second oligonucleotide or an identified reference nucleic acid, such as a target nucleic acid. A region of an oligonucleotide can have a nucleobase sequence that is complementary to a second oligonucleotide or an identified reference nucleic acid, such as a target nucleic acid. The nucleobase sequence of a region or entire length of an oligonucleotide can be at least 50%, at least 60%, at least 70%, at least 80%, at least 85%, at least 90%, at least 95%, or 100% complementary to the second oligonucleotide or nucleic acid, such as a target nucleic acid.

### II. Certain Oligomeric Compounds

Oligomeric compounds may consist of an oligonucleotide (modified or unmodified) and optionally one or more conjugate groups and/or terminal groups. Conjugate groups consist of one or more conjugate moiety and a conjugate linker which links the conjugate moiety to the oligonucleotide. Conjugate groups may be attached to either or both ends of an oligonucleotide and/or at any internal position. In certain embodiments, conjugate groups are attached to the 2'-position of a nucleoside of a modified oligonucleotide. In certain embodiments, conjugate groups that are attached to either or both ends of an oligonucleotide are terminal groups. In certain such embodiments, conjugate groups or terminal groups are attached at the 3' and/or 5'-end of oligonucleotides. In certain such embodiments, conjugate groups (or terminal groups) are attached at the 3'-end of oligonucleotides. In certain embodiments, conjugate groups are attached near the 3'-end of oligonucleotides. In certain embodiments, conjugate groups (or terminal groups) are attached at the 5'-end of oligonucleotides. In certain embodiments, conjugate groups are attached near the 5'-end of oligonucleotides.

Examples of terminal groups include but are not limited to conjugate groups, capping groups, phosphate moieties, protecting groups, modified or unmodified nucleosides, and two or more nucleosides that are independently modified or unmodified.

### A. Certain Conjugate Groups

In certain embodiments, oligonucleotides are covalently attached to one or more conjugate groups. In certain embodiments, conjugate groups modify one or more properties of the attached oligonucleotide, including but not limited to pharmacodynamics, pharmacokinetics, stability, binding, absorption, tissue distribution, cellular distribution, cellular uptake, charge and clearance. In certain embodiments, conjugate groups impart a new property on the attached oligonucleotide, *e.g*., fluorophores or reporter groups that enable detection of the oligonucleotide. Certain conjugate groups and conjugate moieties have been described previously, for example: cholesterol moiety (Letsinger et al., Proc. Natl. Acad. Sci. USA, 1989, 86, 6553-6556), cholic acid (Manoharan et al., Bioorg. Med. Chem. Lett., 1994, 4, 1053-1060), a thioether, *e.g.,* hexyl-S-tritylthiol (Manoharan et al., Ann. N.Y. Acad. Sci., 1992, 660, 306-309; Manoharan et al., Bioorg. Med. Chem. Lett., 1993, 3, 2765-2770), a thiocholesterol (Oberhauser et al., Nucl. Acids Res., 1992, 20, 533-538), an aliphatic chain, *e.g.,* do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid a palmityl moiety (Mishra et al., Biochim. Biophys. Acta, 1995, 1264, 229-237), an octadecylamine or hexylamino-carbonyl-oxycholesterol moiety (Crooke et al., J. Pharmacol. Exp. Ther., 1996, 277, 923-937), a tocopherol group (Nishina et al., Molecular Therapy Nucleic Acids, 2015, 4, e220; and Nishina et al., Molecular Therapy, 2008,16, 734-740), or a GalNAc cluster (*e.g*., WO2014/179620).

### 1. Conjugage Moieties

Conjugate moieties include, without limitation, intercalators, reporter molecules, polyamines, polyamides, peptides, carbohydrates, vitamin moieties, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins, fluorophores, and dyes.

In certain embodiments, a conjugate moiety comprises an active drug substance, for example, aspirin, warfarin, phenylbutazone, ibuprofen, suprofen, fen-bufen, ketoprofen, (*S*)-(+)-pranoprofen, carprofen, dansylsarcosine, 2,3,5-triiodobenzoic acid, fingolimod, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

### 2. Conjugage Linkers

Conjugate moieties are attached to oligonucleotides through conjugate linkers. In certain oligomeric compounds, the conjugate linker is a single chemical bond (i.e., the conjugate moiety is attached directly to an oligonucleotide through a single bond). In certain embodiments, the conjugate linker comprises a chain structure, such as a hydrocarbyl chain, or an oligomer of repeating units such as ethylene glycol, nucleosides, or amino acid units.

In certain embodiments, a conjugate linker comprises one or more groups selected from alkyl, amino, oxo, amide, disulfide, polyethylene glycol, ether, thioether, and hydroxylamino. In certain such embodiments, the conjugate linker comprises groups selected from alkyl, amino, oxo, amide and ether groups. In certain embodiments, the conjugate linker comprises groups selected from alkyl and amide groups. In certain embodiments, the conjugate linker comprises groups selected from alkyl and ether groups. In certain embodiments, the conjugate linker comprises at least one phosphorus moiety. In certain embodiments, the conjugate linker comprises at least one phosphate group. In certain embodiments, the conjugate linker includes at least one neutral linking group.

In certain embodiments, conjugate linkers, including the conjugate linkers described above, are bifunctional linking moieties, *e.g*., those known in the art to be useful for attaching conjugate groups to parent compounds, such as the oligonucleotides provided herein. In general, a bifunctional linking moiety comprises at least two functional groups. One of the functional groups is selected to bind to a particular site on a parent compound and the other is selected to bind to a conjugate group. Examples of functional groups used in a bifunctional linking moiety include but are not limited to electrophiles for reacting with nucleophilic groups and nucleophiles for reacting with electrophilic groups. In certain embodiments, bifunctional linking moieties comprise one or more groups selected from amino, hydroxyl, carboxylic acid, thiol, alkyl, alkenyl, and alkynyl.

Examples of conjugate linkers include but are not limited to pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other conjugate linkers include but are not limited to substituted or unsubstituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

In certain embodiments, conjugate linkers comprise 1-10 linker-nucleosides. In certain embodiments, conjugate linkers comprise 2-5 linker-nucleosides. In certain embodiments, conjugate linkers comprise exactly 3 linker-nucleosides. In certain embodiments, conjugate linkers comprise the TCA motif. In certain embodiments, such linker-nucleosides are modified nucleosides. In certain embodiments such linker-nucleosides comprise a modified sugar moiety. In certain embodiments, linker-nucleosides are unmodified. In certain embodiments, linker-nucleosides comprise an optionally protected heterocyclic base selected from a purine, substituted purine, pyrimidine or substituted pyrimidine. In certain embodiments, a cleavable moiety is a nucleoside selected from uracil, thymine, cytosine, 4-N-benzoylcytosine, 5-methylcytosine, 4-N-benzoyl-5-methylcytosine, adenine, 6-N-benzoyladenine, guanine and 2-N-isobutyrylguanine. It is typically desirable for linker-nucleosides to be cleaved from the oligomeric compound after it reaches a target tissue. Accordingly, linker-nucleosides are typically linked to one another and to the remainder of the oligomeric compound through cleavable bonds. In certain embodimements, such cleavable bonds are phosphodiester bonds.

Herein, linker-nucleosides are not considered to be part of the oligonucleotide. Accordingly, in embodiments in which an oligomeric compound comprises an oligonucleotide consisting of a specified number or range of linked nucleosides and/or a specified percent complementarity to a reference nucleic acid and the oligomeric compound also comprises a conjugate group comprising a conjugate linker comprising linker-nucleosides, those linker-nucleosides are not counted toward the length of the oligonucleotide and are not used in determining the percent complementarity of the oligonucleotide for the reference nucleic acid. For example, an oligomeric compound may comprise (1) a modified oligonucleotide consisting of 8-30 nucleosides and (2) a conjugate group comprising 1-10 linker-nucleosides that are contiguous with the nucleosides of the modified oligonucleotide. The total number of contiguous linked nucleosides in such an oligomeric compound is more than 30. Alternatively, an oligomeric compound may comprise a modified oligonucleotide consisting of 8-30 nucleosides and no conjugate group. The total number of contiguous linked nucleosides in such an oligomeric compound is no more than 30. Unless otherwise indicated conjugate linkers comprise no more than 10 linker-nucleosides. In certain embodiments, conjugate linkers comprise no more than 5 linker-nucleosides. In certain embodiments, conjugate linkers comprise no more than 3 linker-nucleosides. In certain embodiments, conjugate linkers comprise no more than 2 linker-nucleosides. In certain embodiments, conjugate linkers comprise no more than 1 linker-nucleoside.

In certain embodiments, it is desirable for a conjugate group to be cleaved from the oligonucleotide. For example, in certain circumstances oligomeric compounds comprising a particular conjugate moiety are better taken up by a particular cell type, but once the oligomeric compound has been taken up, it is desirable that the conjugate group be cleaved to release the unconjugated or parent oligonucleotide. Thus, certain conjugate linkers may comprise one or more cleavable moieties. In certain embodiments, a cleavable moiety is a cleavable bond. In certain embodiments, a cleavable moiety is a group of atoms comprising at least one cleavable bond. In certain embodiments, a cleavable moiety comprises a group of atoms having one, two, three, four, or more than four cleavable bonds. In certain embodiments, a cleavable moiety is selectively cleaved inside a cell or subcellular compartment, such as a lysosome. In certain embodiments, a cleavable moiety is selectively cleaved by endogenous enzymes, such as nucleases.

In certain embodiments, a cleavable bond is selected from among: an amide, an ester, an ether, one or both esters of a phosphodiester, a phosphate ester, a carbamate, or a disulfide. In certain embodiments, a cleavable bond is one or both of the esters of a phosphodiester. In certain embodiments, a cleavable moiety comprises a phosphate or phosphodiester. In certain embodiments, the cleavable moiety is a phosphate linkage between an oligonucleotide and a conjugate moiety or conjugate group.

In certain embodiments, a cleavable moiety comprises or consists of one or more linker-nucleosides. In certain such embodiments, the one or more linker-nucleosides are linked to one another and/or to the remainder of the oligomeric compound through cleavable bonds. In certain embodiments, such cleavable bonds are unmodified phosphodiester bonds. In certain embodiments, a cleavable moiety is 2'-deoxy nucleoside that is attached to either the 3' or 5'-terminal nucleoside of an oligonucleotide by a phosphate internucleoside linkage and covalently attached to the remainder of the conjugate linker or conjugate moiety by a phosphate or phosphorothioate linkage. In certain such embodiments, the cleavable moiety is 2'-deoxyadenosine.

### III. Duplexed Oligomeric Compounds

Oligomeric compounds described herein comprise an oligonucleotide, having a nucleobase sequence complementary to that of a target nucleic acid. In certain embodiments, an oligomeric compound is paired with a second oligomeric compound to form an oligomeric duplex. Such oligomeric duplexes comprise a first oligomeric compound having a region complementary to a target nucleic acid and a second oligomeric compound having a region complementary to the first oligomeric compound. In certain embodiments, the first oligomeric compound of an oligomeric duplex comprises or consists of (1) a modified or unmodified oligonucleotide and optionally a conjugate group and (2) a second modified or unmodified oligonucleotide and optionally a conjugate group. Either or both oligomeric compounds of an oligomeric duplex may comprise a conjugate group. The oligonucleotides of each oligomeric compound of an oligomeric duplex may include non-complementary overhanging nucleosides.

### IV. Antisense Activity

In certain embodiments, oligomeric compounds and oligomeric duplexes are capable of hybridizing to a target nucleic acid, resulting in at least one antisense activity; such oligomeric compounds and oligomeric duplexes are antisense compounds. In certain embodiments, antisense compounds selectively affect one or more target nucleic acid. Such antisense compounds comprise a nucleobase sequence that hybridizes to one or more target nucleic acid, resulting in one or more desired antisense activity and does not hybridize to one or more non-target nucleic acid or does not hybridize to one or more non-target nucleic acid in such a way that results in significant undesired antisense activity.

In certain antisense activities, hybridization of an antisense compound to a target nucleic acid results in recruitment of a protein that cleaves the target nucleic acid. For example, certain antisense compounds result in RNase H mediated cleavage of the target nucleic acid. RNase H is a cellular endonuclease that cleaves the RNA strand of an RNA:DNA duplex. The DNA in such an RNA:DNA duplex need not be unmodified DNA. In certain embodiments, described herein are antisense compounds that are sufficiently "DNA-like" to elicit RNase H activity. In certain embodiments, one or more non-DNA-like nucleoside in the gap of a gapmer is tolerated.

In certain antisense activities, an antisense compound or a portion of an antisense compound is loaded into an RNA-induced silencing complex (RISC), ultimately resulting in cleavage of the target nucleic acid. For example, certain antisense compounds result in cleavage of the target nucleic acid by Argonaute. Antisense compounds that are loaded into RISC are RNAi compounds. RNAi compounds may be doublestranded (siRNA) or single-stranded (ssRNA).

In certain embodiments, hybridization of an antisense compound to a target nucleic acid does not result in recruitment of a protein that cleaves that target nucleic acid. In certain embodiments, hybridization of the antisense compound to the target nucleic acid results in alteration of splicing of the target nucleic acid. In certain embodiments, hybridization of an antisense compound to a target nucleic acid results in inhibition of a binding interaction between the target nucleic acid and a protein or other nucleic acid. In certain embodiments, hybridization of an antisense compound to a target nucleic acid results in alteration or translation of the target nucleic acid.

Antisense activities may be observed directly or indirectly. In certain embodiments, observation or detection of an antisense activity involves observation or detection of a change in an amount of a target nucleic acid or protein encoded by such target nucleic acid, a change in the ratio of splice variants of a nucleic acid or protein, and/or a phenotypic change in a cell or animal.

### V. Certain Target Nucleic Acids

Oligomeric compounds may comprise or consist of an oligonucleotide comprising a region that is complementary to a target nucleic acid. The target nucleic acid may be an endogenous RNA molecule. The target nucleic acid may encode a protein. The target nucleic acid may be selected from: a mature mRNA and a pre-mRNA, including intronic, exonic and untranslated regions. The target RNA may be a mature mRNA. The target nucleic acid may be a pre-mRNA. The target region may be entirely within an intron. The target region may span an intron/exon junction. The target region may be at least 50% within an intron.

The target nucleic acid may be a non-coding RNA. The target non-coding RNA may be selected from: a long-non-coding RNA, a short non-coding RNA, an intronic RNA molecule, a snoRNA, a scaRNA, a microRNA (including pre-microRNA and mature microRNA), a ribosomal RNA, and promoter directed RNA. The target nucleic acid may be a nucleic acid other than a mature mRNA. The target nucleic acid may be a nucleic acid other than a mature mRNA or a microRNA. The target nucleic acid may be a non-coding RNA other than a microRNA. The target nucleic acid may be a non-coding RNA other than a microRNA or an intronic region of a pre-mRNA. The target nucleic acid may be a long non-coding RNA. The target nucleic acid may be a non-coding RNA associated with splicing of other pre-mRNAs. The target nucleic acid may be a nuclear-retained non-coding RNA.

Oligomeric compounds described herein may becomplementary to a target nucleic acid comprising a single-nucleotide polymorphism (SNP). The oligomeric compound may be capable of modulating expression of one allele of the SNP-containing target nucleic acid to a greater or lesser extent than it modulates another allele. An oligomeric compound may hybridize to a (SNP)-containing target nucleic acid at the single-nucleotide polymorphism site.

Oligomeric compounds may be at least partially complementary to more than one target nucleic acid. For example, oliogomeric compounds described herein may mimic microRNAs, which typically bind to multiple targets.

### A. Complementarity/Mismatches to the Target Nucleic Acid

It is possible to incroduce mismatch bases without eliminating activity. For example, Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL in vitro and in vivo. Furthermore, this oligonucleotide demonstrated potent antitumor activity in vivo. Maher and Dolnick (Nuc. Acid. Res. 16:3341-3358, 1988) tested a series of tandem 14 nucleobase oligonucleotides, and a 28 and 42 nucleobase oligonucleotides comprised of the sequence of two or three of the tandem oligonucleotides, respectively, for their ability to arrest translation of human DHFR in a rabbit reticulocyte assay. Each of the three 14 nucleobase oligonucleotides alone was able to inhibit translation, albeit at a more modest level than the 28 or 42 nucleobase oligonucleotides.

In certain embodiments, oligomeric compounds comprise oligonucleotides that are complementary to the target nucleic acid over the entire length of the oligonucleotide. In certain embodiments, oligonucleotides are 99%, 95%, 90%, 85%, or 80% complementary to the target nucleic acid. Oligonucleotides can be at least 80% complementary to the target nucleic acid over the entire length of the oligonucleotide and comprise a region that is 100% or fully complementary to a target nucleic acid. In certain embodiments, the region of full complementarity is from 6 to 20, 10 to 18, or 18 to 20 nucleobases in length.

In certain embodiments, oligonucleotides comprise one or more mismatched nucleobases relative to the target nucleic acid. In certain embodiments, antisense activity against the target is reduced by such mismatch, but activity against a non-target is reduced by a greater amount. Thus, in certain embodiments selectivity of the oligomeric compound comprising an oligonucleotide is improved. In certain embodiments, the mismatch is specifically positioned within an oligonucleotide having a gapmer motif. In certain embodiments, the mismatch is at position 1, 2, 3, 4, 5, 6, 7, or 8 from the 5'-end of the gap region. In certain embodiments, the mismatch is at position 9, 8, 7, 6, 5, 4, 3, 2, 1 from the 3'-end of the gap region. In certain embodiments, the mismatch is at position 1, 2, 3, or 4 from the 5'-end of the wing region. In certain embodiments, the mismatch is at position 4, 3, 2, or 1 from the 3'-end of the wing region.

### B. C9ORF72

Oligomeric compounds described herein comprise or consist of any oligonucleotide comprising a region that is complementary to a target nucleic acid, wherein the target nucleic acid is C9ORF72. In certain embodiments, C9ORF72 nucleic acid has the sequence set forth in GENBANK Accession No. NM_018325.4 (incorporated herein as SEQ ID NO: 1) or the complement of GENBANK Accession No. NT_008413.18 truncated from nucleobase 27535000 to 27565000 (incorporated herein as SEQ ID NO: 2).

In certain embodiments, contacting a cell with an oligonucleotide complementary to SEQ ID NO: 1 or SEQ ID NO: 2 reduces the amount of C9ORF72 RNA. In certain embodiments, contacting a cell with an oligonucleotide complementary to SEQ ID NO: 1 or SEQ ID NO: 2 reduces the amount of total C9ORF72 protein. In certain embodiments, contacting a cell with an oligomeric compound complementary to SEQ ID NO: 1 or SEQ ID NO: 2 ameliroates one or more symptoms of a neurodegenerative disease, such as anxiety, reduced spatial learning, and memory loss.

### C. Certain Target Nucleic Acids in Certain Tissues

In certain embodiments, oligomeric compounds comprise or consist of an oligonucleotide comprising a region that is complementary to a target nucleic acid, wherein the target nucleic acid is expressed in CNS tissue, including spinal cord and cortex.

### VI. Certain Pharmaceutical Compositions

In certain embodiments, described herein are pharmaceutical compositions comprising one or more oligomeric compounds or a salt thereof. In certain embodiments, the pharmaceutical composition comprises a pharmaceutically acceptable diluent or carrier. In certain embodiments, a pharmaceutical composition comprises a sterile saline solution and one or more oligomeric compound. In certain embodiments, a pharmaceutical composition consists of a sterile saline solution and one or more oligomeric compound. In certain embodiments, the sterile saline is pharmaceutical grade saline. In certain embodiments, a pharmaceutical composition comprises one or more oligomeric compound and sterile water. In certain embodiments, a pharmaceutical composition consists of one oligomeric compound and sterile water. In certain embodiments, the sterile water is pharmaceutical grade water. In certain embodiments, a pharmaceutical composition comprises one or more oligomeric compound and phosphate-buffered saline (PBS). In certain embodiments, a pharmaceutical composition consists of one or more oligomeric compound and sterile PBS. In certain embodiments, the sterile PBS is pharmaceutical grade PBS.

In certain embodiments, pharmaceutical compositions comprise one or more oligomeric compound and one or more excipients. In certain embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, oligomeric compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

In certain embodiments, pharmaceutical compositions comprising an oligomeric compound encompass any pharmaceutically acceptable salts of the oligomeric compound, esters of the oligomeric compound, or salts of such esters. In certain embodiments, pharmaceutical compositions comprising oligomeric compounds comprising one or more oligonucleotide, upon administration to an animal, including a human, are capable of providing (directly or indirectly) the biologically active metabolite or residue thereof. Accordingly, for example, the disclosure is also drawn to pharmaceutically acceptable salts of oligomeric compounds, prodrugs, pharmaceutically acceptable salts of such prodrugs, and other bioequivalents. Suitable pharmaceutically acceptable salts include, but are not limited to, sodium and potassium salts. In certain embodiments, prodrugs comprise one or more conjugate group attached to an oligonucleotide, wherein the conjugate group is cleaved by endogenous nucleases within the body.

Lipid moieties have been used in nucleic acid therapies in a variety of methods. In certain such methods, the nucleic acid, such as an oligomeric compound, is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions comprise a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, pharmaceutical compositions comprise one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, pharmaceutical compositions comprise a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80^{™} and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80^{™}; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, pharmaceutical compositions are prepared for oral administration. In certain embodiments, pharmaceutical compositions are prepared for buccal administration. In certain embodiments, a pharmaceutical composition is prepared for administration by injection (e.g., intravenous, subcutaneous, intramuscular, intrathecal, intracerebroventricular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain.

### Nonlimiting disclosure

While certain compounds, compositions and methods described herein have been described with specificity in accordance with certain embodiments, the following examples serve only to illustrate the compounds described herein and are not intended to limit the same.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH in place of one 2'-H of DNA) or as an RNA having a modified base (thymine (methylated uracil) in place of a uracil of RNA). Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified nucleobases, such as "AT^{m}CGAUCG," wherein ^{m}C indicates a cytosine base comprising a methyl group at the 5-position.

Certain compounds described herein (e.g., modified oligonucleotides) have one or more asymmetric center and thus give rise to enantiomers, diastereomers, and other stereoisomeric configurations that may be defined, in terms of absolute stereochemistry, as (R) or (S), as α or β such as for sugar anomers, or as (D) or (L), such as for amino acids, etc. Included in the compounds provided herein are all such possible isomers, including their racemic and optically pure forms, unless specified otherwise. Likewise, all cis- and trans-isomers and tautomeric forms are also included unless otherwise indicated. Unless otherwise indicated, compounds described herein are intended to include corresponding salt forms.

### EXAMPLES

The following examples illustrate certain embodiments of the present disclosure and are not limiting. Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

### Example 1: Modified oligonucleotides targeting human C9ORF72

The modified oligonucleotide in the table below is a MOE gapmer. The central gap segment of the gapmer contains 2'-deoxynucleosides and is flanked by wing segments on both the 5' end and on the 3' end containing nucleosides that each comprise a 2'-MOE group. The specific motif of the gapmer is listed in table below, represented by three numbers separated by hyphens. The numbers represent the number of nucleosides in the 5'-wing, the gap, and the 3'-wing, respectively. All cytosine residues throughout the oligonucleotide are 5-methylcytosines. The internucleoside linkages for the gapmer are mixed phosphorothioate and phosphodiester linkages. The internucleoside linkages for the gapmer are presented in the Linkage column, where 'o' indicates a phosphodiester linkage and's' indicates a phosphorothioate linkage.

The modified oligonucleotide listed in the table below is targeted to the human C9ORF72 genomic sequence, designated herein as SEQ ID NO: 2 (the complement of GENBANK Accession No. NT_008413.18 truncated from nucleosides 27535000 to 27565000). "Start site" indicates the 5'-most nucleoside to which the gapmer is targeted in the human genomic sequence. "Stop site" indicates the 3'-most nucleoside to which the gapmer is targeted human genomic sequence.

**Table 1**

| **Modified oligonucleotides targeting human C9ORF72** | | | | | | |
|---|---|---|---|---|---|---|
| Compound No | Sequence | Linkage | Start Site SEQ ID NO: 2 | Stop Site SEQ ID NO: 2 | Motif | SEQ ID NO |
| 672681 | GCCCCTAGCGCGCGACTC | sooosssssssssooss | 1444 | 1461 | 5-8-5 | 6 |

### Example 2: In vitro dose response inhibition of human pathogenic C9ORF72 RNA in HepG2 cells with 672681

Compound No. 672681 was tested at various doses in HepG2 cells. Cells were plated at a density of 20,000 cells per well and transfected using electroporation with 0.11 µM, 0.33 µM, 1.00 µM, or 3.00 µM concentrations of modified oligonucleotide. After a treatment period of approximately 16 hours, RNA was isolated from the cells and C9ORF72 mRNA levels were measured by quantitative real-time PCR using human primer probe set RTS3905 (forward primer: GGGTCTAGCAAGAGCAGGTG, designated herein as SEQ ID NO: 3; reverse primer: GTCTTGGCAACAGCTGGAGAT, designated herein as SEQ ID NO: 4; probe: TGATGTCGACTCTTTGCCCACCGC, designated herein as SEQ ID NO: 5 - a TAQ-man primer probe set). Primer probe set RTS3905 was used to measure the pathogenic C9ORF72 RNA, which is the product of a pre-mRNA containing a hexanucleotide repeat. C9ORF72 mRNA levels were adjusted according to total RNA content, as measured by RIBOGREEN^{®}. Results are presented in Table 2 below as percent inhibition of human C9ORF72 mRNA levels, relative to untreated control cells.

**Table 2**

| **Percent inhibition of the pathogenic C9ORF72 RNA in HepG2 cells** | | | | |
|---|---|---|---|---|
| Compound No | 0.11 µM | 0.33 µM | 1.00 µM | 3.00 µM |
| 672681 | 27 | 42 | 63 | 87 |

### Example 3: Inhibition of human C9ORF72 RNA in transgenic mice

Inhibition of C9ORF72 RNA was tested using Compopund No. 672681 in a BAC transgenic mouse line, designated herein as C9B183. The C9B183 mouse line expresses a truncated human C9ORF72 gene comprising exons 1-5. The truncated human C9ORF72 gene of the C9B183 mouse line contains 450 hexanucleotide repeats.

3-month old C9B183 mice each received a single intra cerebroventricular (ICV) stereotactic injection into the right ventricle of 350 µg of either Compound No. 672681 or a control oligonucleotide that does not target human C9ORF72 in 10 µL total volume of PBS. Each treatment group consisted of six mice. Two weeks following oligonucleotide treatment, the mice were sacrificed and spinal cord and cortex tissues were collected from each mouse. Total RNA from each tissue was isolated with TRIZOL (Invitrogen, Carlsbad, CA) and first-strand cDNA was synthesized using the SuperScript III First-strand synthesis kit (Invitrogen, Carlsbad, CA). RT-qPCR of human pathogenic C9ORF72 RNA was performed using primer probe set RTS3905 (see Example 2) and normalized to GAPDH. The average results for each treatment group are presented in the table below as normalized percent inhibition of human C9ORF72 RNA relative to the control treated mice.

**Table 3**

| **Percent inhibition of human pathogenic C9ORF72 RNA *in vivo*** | | |
|---|---|---|
| Oligonucleotide | Human C9OR F72 RNA (%) | |
| | Cortex | Spinal Cord |
| Control | 100 | 100 |
| Isis No. 672681 | 23 | 12 |

### Example 4: Behavioral effects of modified oligonucleotide-mediated inhibition of human C9ORF72 RNA

Nine month old wild type and C9B183 mice each received a single ICV stereotactic injection into the right ventricle of 350 µg of either Isis No. 672681 or a control oligonucleotide that does not target human C9ORF72 in 10 µL total volume of PBS. Each treatment group consisted of ten to thirteen mice. At 12 months of age and 15 months of age, the anxiety-related and cognition-related behaviors described below were assessed, except for the radial arm maze, which was assessed only at 12 months.

### Marble Burying

Mice were placed individually in a standard mouse cage containing bedding that is 5 cm in depth, with 20 small marbles arranged in 4 evenly spaced rows of 5 on top of the bedding material. After 20 minutes, mice were removed and the number of marbles buried was determined (marbles that were at least 2/3 covered by bedding were counted as buried). Increased marble burying is associated with increased anxiety-related behavior. The results are presented in the table below as the average percentage of marbles buried for each treatment group.

### Elevated Plus Maze

The plus maze apparatus had four arms (5×30 cm) at right angles to each other and was elevated 30 cm from the floor. Two of the arms had 16 cm high walls (enclosed arms) and two arms had a 0.5 cm lip, but no walls (open arms). Mice were placed onto the center of the maze and allowed free access to all four arms for 5 minutes. Behavior was recorded using a camera mounted above the apparatus. An increased percentage of time spent on the enclosed arms is associated with increased anxiety-related behavior. The results are presented in the table below as the average percentage of time spent in the open arms for each treatment group.

### Barnes Maze

An opaque Plexiglas disc 75 cm in diameter and elevated 58 cm above the floor on a tripod was used as the Barnes Maze. Twenty holes, 5 cm in diameter each, were located 5 cm from the perimeter, and a black Plexiglas escape box (19 × 8 × 7 cm) was placed under one of the holes. Distinct spatial cues were located around the maze and were kept constant throughout the study. On the first day of testing, a training session was performed, in which the mouse was placed in the escape box for five minutes. One minute later, the first session was started. At the beginning of each session, the mouse was placed in the middle of the platform in a 10 cm high cylindrical black start chamber. After 10 seconds, the start chamber was removed, a light (400 lux) was turned on, and the mouse was allowed to freely explore the maze. The session ended when the mouse entered the escape tunnel or after 3 minutes had elapsed. When the mouse entered the escape tunnel, the light was turned off and the mouse remained in the dark for one minute. When the mouse did not enter the tunnel by itself it was gently put in the escape box for one minute. The tunnel was always located underneath the same hole (stable within the spatial environment), which was randomly determined for each mouse. Mice were tested once a day for 9 days. The number of errors in finding the escape chamber one each day of testing was tabulated for each mouse. The results are presented in the table below as the average number of errors for each treatment group over days 7 through 9 of testing. The Barnes maze is a measure of spatial learning and memory.

### Radial Arm Maze

Prior to the start of the study, all animals underwent a 5 day period of food restriction wherein their body weights were reduced to 90% of the original weight by restricting available food to 2 grams of chow per mouse. Once each animal reached the target weight of 90% of original body weight, food rations were adjusted accordingly to this weight. The purpose of the food deprivation was to establish food as a motivator. Next, mice were trained daily on an elevated, 8 arm-radial arm maze, with a circular recessed food cup located at the end of each arm. The first 3 days served as a familiarization period for the mice. On the first day, each mouse was placed on the maze for 10 minutes with no food present. On the next two days, food was scattered on the center platform and over the entire length of four of the arms of the maze. The four arms containing food were distributed randomly throughout the maze (e.g., arms 1, 4, 5, and 7 were baited with food). After completing the familiarization phase, food pellets were placed only in the food cups at the end of four designated arms. Each mouse remained on the maze until it found the four food cups or until 10 minutes had elapsed. During each trial, the number and order of arms entered by each mouse was recorded. Entries into arms that had never contained food following the familiarization phase were considered errors in reference memory. Re-entering a previously entered arm was considered an error in working memory. The results are presented in the table below as the average total number of errors for each treatment group on the indicated day of testing.

**Table 4**

| **Behavioral effects of modified oligonucleotide-mediated inhibition of human C9ORF72 RNA in mice** | | | | | | | | | |
|---|---|---|---|---|---|---|---|---|---|
| Group | Marble Burying (% buried) | | Elevated Plus Maze (% time on open arms) | | Barnes Maze (No. of errors) | | Radial Arm Maze (No. errors) | | |
| | 12 months | 15 months | 12 months | 15 months | 12 months | 15 months | Day 8 | Day 9 | Day 10 |
| WT; control | 51 | 33 | 26.4 | 19.0 | 13.0 | 14.3 | 4.1 | 3.7 | 2.3 |
| WT; Isis No. 672681 | 50 | 38 | 24.5 | 17.3 | 12.2 | 15.9 | 4.0 | 3.8 | 2.6 |
| C9B183; control | 68 | 63 | 14.4 | 7.7 | 20.7 | 23.5 | 6.1 | 6.3 | 6.7 |
| C9B183; Isis No. 672681 | 48 | 39 | 23.6 | 19.9 | 16.7 | 18.4 | 4.9 | 4.9 | 4.5 |

## Claims

1. An oligomeric compound comprising a modified oligonucleotide for use in ameliorating or preventing at least one symptom of a neurodegenerative disease, wherein the modified oligonucleotide consists of 12 to 30 linked nucleosides, wherein the modified oligonucleotide has a nucleobase sequence that is at least 90% complementary to a C9ORF72 nucleic acid, wherein the modified oligonucleotide has a nucleobase sequence comprising at least 12, at least 13, at least 14, at least 15, at least 16, at least 17, or 18 contiguous nucleobases of SEQ ID NO: 6, and wherein the at least one symptom is any of anxiety, reduced spatial learning, and memory loss.

2. The oligomeric compound for use according to claim 1, wherein the neurodegenerative disease is any of amyotrophic lateral sclerosis (ALS), frontotemporal dementia (FTD), corticalbasal degeneration syndrome (CBD), atypical Parkinsonian syndrome, and olivopontocerellar degeneration (OPCD).

3. The oligomeric compound for use according to claim 1 or claim 2, wherein the modified oligonucleotide has a nucleobase sequence that is at least 90% complementary, at least 95% complementary, or 100% complementary to the nucleobase sequence of SEQ ID NO: 1 or 2, when measured across the entire nucleobase sequence of the modified oligonucleotide.

4. The oligomeric compound for use according to any of claims 1-3, wherein:
a. the oligomeric compound is administered prior to the detection of the at least one symptom;
b. the amelioration is the slowing of progression of at least one symptom;
c. the amelioration is the delay of onset of at least one symptom;
d. the amelioration is the reduction of severity of at least one symptom; or
e. the amelioration is the reduction of frequency of at least one symptom.

5. The oligomeric compound for use according to any of claims 1-4, wherein:
a. the amount of total C9ORF72 RNA is reduced or the amount of pathogenic C9ORF72 RNA is reduced; and/or
b. the amount of total C9ORF72 protein is reduced.

6. The oligomeric compound for use according to any of claims 1-5, wherein the oligomeric compound is single-stranded.

7. The oligomeric compound for use according to any of claim 1-6, wherein the modified oligonucleotide comprises at least one modified nucleoside.

8. The oligomeric compound for use according to claim 7, wherein:
a. the modified oligonucleotide comprises at least one modified nucleoside comprising a modified sugar moiety, optionally wherein the modified oligonucleotide comprises at least one modified nucleoside comprising a bicyclic sugar moiety, preferably wherein the modified oligonucleotide comprises at least one modified nucleoside comprising a bicyclic sugar moiety having a 2'-4' bridge, wherein the 2-4' bridge is selected from -O-CH₂-; -O-CH₂-CH₂; and -O-CH(CH₃)-; and/or
b. the modified oligonucleotide comprises at least one modified nucleoside comprising a modified non-bicyclic sugar moiety, optionally wherein the modified oligonucleotide comprises at least one modified nucleoside comprising a non-bicyclic sugar moiety comprising a 2'-MOE or 2'-OMe.

9. The oligomeric compound for use according to claim 8, wherein the modified oligonucleotide comprises at least one modified nucleoside comprising a sugar surrogate, optionally wherein the modified oligonucleotide comprises at least one modified nucleoside comprising a sugar surrogate selected from a morpholino, a PNA, a F-HNA, a THP, or a modified THP.

10. The oligomeric compound for use according to any of claims 1-9, wherein the modified oligonucleotide has a sugar motif comprising:
a 5'-region consisting of 1-5 linked 5'-nucleosides;
a central region consisting of 6-10 linked central region nucleosides; and
a 3'-region consisting of 1-5 linked 3'-region nucleosides; wherein each of the 5'-region nucleosides and each of the 3'-region nucleosides comprises a modified sugar moiety and each of the central region nucleosides comprises an unmodified DNA sugar moiety.

11. The oligomeric compound for use according to any of claims 1-10, wherein:
a. the modified oligonucleotide comprises at least one modified internucleoside linkage, optionally wherein the at least one modified internucleoside linkage is a phosphorothioate internucleoside linkage, preferably wherein the modified oligonucleotide comprises at least one phosphodiester internucleoside linkage;
b. each internucleoside linkage of the modified oligonucleotide is a modified internucleoside linkage, optionally wherein at least one or each modified internucleoside linkage is a phosphorothioate internucleoside linkage; or
c. each internucleoside linkage is either a phosphodiester internucleoside linkage or a phosphorothioate internucleoside linkage.

12. The oligomeric compound for use according to any of claims 1-11, wherein the modified oligonucleotide comprises at least one modified nucleobase, optionally wherein the modified nucleobase is a 5-methylcytosine, and/or wherein each nucleobase of each nucleoside of the modified oligonucleotide is either an unmodified nucleobase or is a 5-methylcytosine.

13. The oligomeric compound for use according to any of claims 1-12, wherein the oligomeric compound comprises a conjugate group; and/or the oligomeric compound is paired with a second oligomeric compound to form a duplex.

14. The oligomeric compound for use according to any of claims 1-13, wherein the modified oligonucleotide of the compound is a sodium salt.

15. A pharmaceutical composition comprising the compound of any of claims 1-14 and a pharmaceutically acceptable diluent or carrier for use in ameliorating or preventing at least one symptom of a neurodegenerative disease, wherein the at least one symptom is any of anxiety, reduced spatial learning, and memory loss, optionally wherein the pharmaceutically acceptable diluent is phosphate-buffered saline.

## Patentansprüche

1. Oligomerverbindung, umfassend ein modifiziertes Oligonukleotid, zur Verwendung beim Verbessern oder Vorbeugen wenigstens eines Symptoms einer neurodegenerativen Erkrankung, wobei das modifizierte Oligonukleotid aus 12 bis 30 verknüpften Nukleosiden besteht, wobei das modifizierte Oligonukleotid eine Nukleobasensequenz aufweist, die wenigstens 90% komplementär zu einer C9ORF72-Nukleinsäure ist, wobei das modifizierte Oligonukleotid eine Nukleobasensequenz aufweist, die wenigstens 12, wenigstens 13, wenigstens 14, wenigstens 15, wenigstens 16, wenigstens 17 oder 18 zusammenhängende Nukleobasen von SEQ ID NO: 6 umfasst und wobei es sich bei dem wenigstens einen Symptom um eines aus Angstzustand, vermindertem räumlichen Lernen und Gedächtnisverlust handelt.

2. Oligomerverbindung zur Verwendung nach Anspruch 1, wobei es sich bei der neurodegenerativen Erkrankung um eine aus amyotropher Lateralsklerose (ALS), frontotemporaler Demenz (FTD), corticobasalem Degenerationssyndrom (CBD), atypischem Parkinson-Syndrom und olivopontozerellärer Degeneration (OPCD) handelt.

3. Oligomerverbindung zur Verwendung nach Anspruch 1 oder Anspruch 2, wobei das modifizierte Oligonukleotid eine Nukleobasensequenz aufweist, die beim Messen über die gesamte Nukleobasensequenz des modifizierten Oligonukleotids wenigstens 90% komplementär, wenigstens 95% komplementär oder 100% komplementär zur Nukleobasensequenz unter SEQ ID NO: 1 oder 2 ist.

4. Oligomerverbindung zur Verwendung nach einem der Ansprüche 1-3, wobei:
a. die Oligomerverbindung vor dem Nachweis des wenigstens einen Symptoms verabreicht wird;
b. es sich bei der Verbesserung um das Verlangsamen des Fortschreitens wenigstens eines Symptoms handelt;
c. es sich bei der Verbesserung um die Verzögerung des Ausbruchs wenigstens eines Symptoms handelt;
d. es sich bei der Verbesserung um die Verringerung der Schwere wenigstens eines Symptoms handelt; oder
e. es sich bei der Verbesserung um die Verringerung der Häufigkeit wenigstens eines Symptoms handelt.

5. Oligomerverbindung zur Verwendung nach einem der Ansprüche 1-4, wobei:
a. die Menge an C9ORF72-Gesamt-RNA oder die Menge an pathogener C9ORF72-RNA verringert wird; und/oder
b. die Menge an C9ORF72-Gesamtprotein verringert wird.

6. Oligomerverbindung zur Verwendung nach einem der Ansprüche 1-5, wobei die Oligomerverbindung einzelsträngig ist.

7. Oligomerverbindung zur Verwendung nach einem der Ansprüche 1-6, wobei das modifizierte Oligonukleotid wenigstens ein modifiziertes Nukleosid umfasst.

8. Oligomerverbindung zur Verwendung nach Anspruch 7, wobei:
a. das modifizierte Oligonukleotid wenigstens ein modifiziertes Nukleosid umfasst, das eine modifizierte Zuckergruppierung umfasst, gegebenenfalls wobei das modifizierte Oligonukleotid wenigstens ein modifiziertes Nukleosid umfasst, das eine bicyclische Zuckergruppierung umfasst, bevorzugt wobei das modifizierte Oligonukleotid wenigstens ein modifiziertes Nukleosid umfasst, das eine bicyclische Zuckergruppierung mit einer 2'-4'-Brücke umfasst, wobei die 2'-4'-Brücke ausgewählt ist aus -O-CH₂-; -O-CH₂-CH₂; und-O-CH(CH₃)-; und/oder
b. das modifizierte Oligonukleotid wenigstens ein modifiziertes Nukleosid umfasst, das eine modifizierte nicht bicyclische Zuckergruppierung umfasst, gegebenenfalls wobei das modifizierte Oligonukleotid wenigstens ein modifiziertes Nukleosid umfasst, das eine nicht bicyclische Zuckergruppierung umfasst, die ein 2'-MOE oder 2'-OMe umfasst.

9. Oligomerverbindung zur Verwendung nach Anspruch 8, wobei das modifizierte Oligonukleotid wenigstens ein modifiziertes Nukleosid umfasst, das ein Zuckersurrogat umfasst, gegebenenfalls wobei das modifizierte Oligonukleotid wenigstens ein modifiziertes Nukleosid umfasst, das ein Zuckersurrogat ausgewählt aus einem Morpholino, einer PNA, einer F-HNA, einem THP or einem modifizierten THP umfasst.

10. Oligomerverbindung zur Verwendung nach einem der Ansprüche 1-9, wobei das modifizierte Oligonukleotid ein Zuckermotiv aufweist, umfassend:
eine 5'-Region, die aus 1-5 verknüpften 5'-Nukleosiden besteht;
eine zentrale Region, die aus 6-10 verknüpften Zentralregion-Nukleosiden besteht; und
eine 3'-Region, die aus 1-5 verknüpften 3'-Region-Nukleosiden besteht; die 5'-Region-Nukleoside und die 3'-Region-Nukleoside jeweils eine modifizierte Zuckergruppierung umfassen und die Zentralregion-Nukleoside jeweils eine unmodifizierte DNA-Zuckergruppierung umfassen.

11. Oligomerverbindung zur Verwendung nach einem der Ansprüche 1-10, wobei:
a. das modifizierte Oligonukleotid wenigstens eine modifizierte Internukleosidverknüpfung umfasst, gegebenenfalls wobei es sich bei der wenigstens einen modifizierten Internukleosidverknüpfung um eine Phosphorthioat-Internukleosidverknüpfung handelt, vorzugsweise wobei das modifizierte Oligonukleotid wenigstens eine Phosphodiester-Internukleosidverknüpfung umfasst;
b. es sich bei den Internukleosidverknüpfungen des modifizierten Oligonukleotids jeweils um eine modifizierte Internukleosidverknüpfung handelt, gegebenenfalls wobei es sich bei wenigstens einer oder jeder modifizierten Internukleosidverknüpfung um eine Phosphorthioat-Internukleosidverknüpfung handelt; oder
c. es sich bei den Internukleosidverknüpfungen jeweils um entweder eine Phosphodiester-Internukleosidverknüpfung oder eine Phosphorthioat-Internukleosidverknüpfung handelt.

12. Oligomerverbindung zur Verwendung nach einem der Ansprüche 1-11, wobei das modifizierte Oligonukleotid wenigstens eine modifizierte Nukleobase umfasst, gegebenenfalls wobei es sich bei der modifizierten Nukleobase um ein 5-Methylcytosin handelt und/oder wobei es sich bei jeder Nukleobase jedes Nukleosids des modifizierten Oligonukleotids um entweder eine unmodifizierte Nukleobase oder ein 5-Methylcytosin handelt.

13. Oligomerverbindung zur Verwendung nach einem der Ansprüche 1-12, wobei die Oligomerverbindung eine Konjugatgruppe umfasst; und/oder die Oligomerverbindung mit einer zweiten Oligomerverbindung unter Ausbildung eines Duplex gepaart ist.

14. Oligomerverbindung zur Verwendung nach einem der Ansprüche 1-13, wobei es sich bei dem modifizierten Oligonukleotid der Verbindung um ein Natriumsalz handelt.

15. Pharmazeutische Zusammensetzung, umfassend die Verbindung nach einem der Ansprüche 1-14 und ein pharmazeutisch unbedenkliches Verdünnungs- oder Trägermittel, zur Verwendung beim Verbessern oder Vorbeugen wenigstens eines Symptoms einer neurodegenerativen Erkrankung, wobei es sich bei dem wenigstens einen Symptom um eines aus Angstzustand, vermindertem räumlichen Lernen und Gedächtnisverlust handelt, gegebenenfalls wobei es sich bei dem pharmazeutisch unbedenklichen Verdünnungsmittel um phosphatgepufferte Salzlösung handelt.

## Revendications

1. Composé oligomère comprenant un oligonucléotide modifié pour une utilisation dans l'amélioration ou la prévention d'au moins un symptôme d'une maladie neurodégénérative, dans lequel l'oligonucléotide modifié consiste en 12 à 30 nucléosides liés, dans lequel l'oligonucléotide modifié a une séquence de nucléobases qui est complémentaire à au moins 90 % d'un acide nucléique de C9ORF72, dans lequel l'oligonucléotide modifié a une séquence de nucléobases comprenant au moins 12, au moins 13, au moins 14, au moins 15, au moins 16, au moins 17 ou 18 nucléobases contiguës de SEQ ID NO : 6, et dans lequel l'au moins un symptôme est l'un quelconque de l'anxiété, d'une réduction de l'apprentissage spatial et d'une perte de mémoire.

2. Composé oligomère pour une utilisation selon la revendication 1, la maladie neurodégénérative étant l'un quelconque parmi la sclérose latérale amyotrophique (SLA), la démence frontotemporale (DFT), le syndrome de dégénérescence cortico-basale (SDCB), le syndrome parkinsonien atypique et la dégénérescence olivo-pontocérébelleuse (DOPC).

3. Composé oligomère pour une utilisation selon la revendication 1 ou la revendication 2, dans lequel l'oligonucléotide modifié a une séquence de nucléobases qui est complémentaire à au moins 90 %, complémentaire à au moins 95 % ou complémentaire à au moins 100 % de la séquence de nucléobases de SEQ ID NO : 1 ou 2, la mesure étant effectuée sur la totalité de la séquence de nucléobases de l'oligonucléotide modifié.

4. Composé oligomère pour une utilisation selon l'une quelconque des revendications 1 à 3,
a. le composé oligomère étant administré avant la détection de l'au moins un symptôme ;
b. l'amélioration étant le ralentissement de la progression d'au moins un symptôme ;
c. l'amélioration étant le retard d'apparition d'au moins un symptôme ;
d. l'amélioration étant la réduction de la gravité d'au moins un symptôme ; ou
e. l'amélioration étant la réduction de la fréquence d'au moins un symptôme.

5. Composé oligomère pour une utilisation selon l'une quelconque des revendications 1 à 4, dans lequel :
a. la quantité d'ARN de C9ORF72 total est réduite ou la quantité d'ARN de C9ORF72 pathogène est réduite ; et/ou
b. la quantité de la protéine C9ORF72 totale est réduite.

6. Composé oligomère pour une utilisation selon l'une quelconque des revendications 1 à 5, le composé oligomère étant simple brin.

7. Composé oligomère pour une utilisation selon l'une quelconque des revendications 1 à 6, dans lequel l'oligonucléotide modifié comprend au moins un nucléoside modifié.

8. Composé oligomère pour une utilisation selon la revendication 7, dans lequel :
a. l'oligonucléotide modifié comprend au moins un nucléoside modifié comprenant une fraction sucre modifiée, éventuellement dans lequel l'oligonucléotide modifié comprend au moins un nucléoside modifié comprenant une fraction sucre bicyclique, de préférence dans lequel l'oligonucléotide modifié comprend au moins un nucléoside modifié comprenant une fraction sucre bicyclique ayant un pont 2',4', le pont 2',4' étant choisi parmi -O-CH₂- ; -O-CH₂-CH₂ ; et -O-CH(CH₃)- ; et/ou
b. l'oligonucléotide modifié comprend au moins un nucléoside modifié comprenant une fraction sucre non bicyclique modifiée, éventuellement dans lequel l'oligonucléotide modifié comprend au moins un nucléoside modifié comprenant une fraction sucre non bicyclique comprenant un 2'-MOE ou un 2'-OMe.

9. Composé oligomère pour une utilisation selon la revendication 8, dans lequel l'oligonucléotide modifié comprend au moins un nucléoside modifié comprenant un succédané de sucre, éventuellement dans lequel l'oligonucléotide modifié comprend au moins un nucléoside modifié comprenant un succédané de sucre choisi parmi un morpholino, un PNA, un F-HNA, un THP ou un THP modifié.

10. Composé oligomère pour une utilisation selon l'une quelconque des revendications 1 à 9, dans lequel l'oligonucléotide modifié a un motif sucre comprenant :
une région en 5' consistant en 1 à 5 nucléosides en 5' liés ;
une région centrale consistant en 6 à 10 nucléosides liés de région centrale ; et
une région en 3' consistant en 1 à 5 nucléosides liés de région en 3' ; chacun des nucléosides de région en 5' et chacun des nucléosides de région en 3' comprenant une fraction sucre modifiée et chacun des nucléosides de région centrale comprenant une fraction ADN sucre non modifiée.

11. Composé oligomère pour une utilisation selon l'une quelconque des revendications 1 à 10, dans lequel :
a. l'oligonucléotide modifié comprend au moins une liaison internucléosidique modifiée, éventuellement dans lequel l'au moins une liaison internucléosidique modifiée est une liaison internucléosidique phosphorothioate, de préférence dans laquelle l'oligonucléotide modifié comprend au moins une liaison internucléosidique phosphodiester ;
b. chaque liaison internucléosidique de l'oligonucléotide modifié est une liaison internucléosidique modifiée, éventuellement dans lequel au moins une ou chaque liaison internucléosidique modifiée est une liaison internucléosidique phosphorothioate ; ou
c. chaque liaison internucléosidique est une liaison internucléosidique phosphodiester ou une liaison internucléosidique phosphorothioate.

12. Composé oligomère pour une utilisation selon l'une quelconque des revendications 1 à 11, dans lequel l'oligonucléotide modifié comprend au moins une nucléobase modifiée, éventuellement dans lequel la nucléobase modifiée est une 5-méthylcytosine, et/ou dans lequel chaque nucléobase de chaque nucléoside de l'oligonucléotide modifié est une nucléobase non modifiée ou une 5-méthylcytosine.

13. Composé oligomère pour une utilisation selon l'une quelconque des revendications 1 à 12, le composé oligomère comprenant un groupe conjugué ; et/ou le composé oligomère étant apparié à un second composé oligomère pour former un duplex.

14. Composé oligomère pour une utilisation selon l'une quelconque des revendications 1 à 13, dans lequel l'oligonucléotide modifié du composé est un sel de sodium.

15. Composition pharmaceutique comprenant le composé selon l'une quelconque des revendications 1 à 14 et un véhicule ou un excipient pharmaceutiquement acceptable pour une utilisation dans l'amélioration ou la prévention d'au moins un symptôme d'une maladie neurodégénérative, l'au moins un symptôme étant l'un quelconque parmi l'anxiété, la réduction de l'apprentissage spatial et la perte de mémoire, éventuellement dans laquelle le diluant pharmaceutiquement acceptable est une solution salée tamponnée par un tampon phosphate.


### SEQUENCE LISTING

<110> Ionis Pharmaceuticals, Inc. Ludwig Institute for Cancer Research
<120> METHODS FOR REDUCING C9ORF72 EXPRESSION
<130> BIOL0288WO
<160> 6
<150> 62/322,146 <151> 2016-04-13
<170> PatentIn version 3.5
<210> 1
   <211> 3261
   <212> DNA
   <213> Homo sapiens
<400> 1
   gggcggggct gcggttgcgg tgcctgcgcc cgcggcggcg gaggcgcagg cggtggcgag      60
   tggatatctc cggagcattt ggataatgtg acagttggaa tgcagtgatg tcgactcttt     120
   gcccaccgcc atctccagct gttgccaaga cagagattgc tttaagtggc aaatcacctt     180
   tattagcagc tacttttgct tactgggaca atattcttgg tcctagagta aggcacattt     240
   gggctccaaa gacagaacag gtacttctca gtgatggaga aataactttt cttgccaacc     300
   acactctaaa tggagaaatc cttcgaaatg cagagagtgg tgctatagat gtaaagtttt     360
   ttgtcttgtc tgaaaaggga gtgattattg tttcattaat ctttgatgga aactggaatg     420
   gggatcgcag cacatatgga ctatcaatta tacttccaca gacagaactt agtttctacc     480
   tcccacttca tagagtgtgt gttgatagat taacacatat aatccggaaa ggaagaatat     540
   ggatgcataa ggaaagacaa gaaaatgtcc agaagattat cttagaaggc acagagagaa     600
   tggaagatca gggtcagagt attattccaa tgcttactgg agaagtgatt cctgtaatgg     660
   aactgctttc atctatgaaa tcacacagtg ttcctgaaga aatagatata gctgatacag     720
   tactcaatga tgatgatatt ggtgacagct gtcatgaagg ctttcttctc aatgccatca     780
   gctcacactt gcaaacctgt ggctgttccg ttgtagtagg tagcagtgca gagaaagtaa     840
   ataagatagt cagaacatta tgcctttttc tgactccagc agagagaaaa tgctccaggt     900
   tatgtgaagc agaatcatca tttaaatatg agtcagggct ctttgtacaa ggcctgctaa     960
   aggattcaac tggaagcttt gtgctgcctt tccggcaagt catgtatgct ccatatccca    1020
   ccacacacat agatgtggat gtcaatactg tgaagcagat gccaccctgt catgaacata    1080
   tttataatca gcgtagatac atgagatccg agctgacagc cttctggaga gccacttcag    1140
   aagaagacat ggctcaggat acgatcatct acactgacga aagctttact cctgatttga    1200
   atatttttca agatgtctta cacagagaca ctctagtgaa agccttcctg gatcaggtct    1260
   ttcagctgaa acctggctta tctctcagaa gtactttcct tgcacagttt ctacttgtcc 1320
   ttcacagaaa agccttgaca ctaataaaat atatagaaga cgatacgcag aagggaaaaa 1380
   agccctttaa atctcttcgg aacctgaaga tagaccttga tttaacagca gagggcgatc 1440
   ttaacataat aatggctctg gctgagaaaa ttaaaccagg cctacactct tttatctttg 1500
   gaagaccttt ctacactagt gtgcaagaac gagatgttct aatgactttt taaatgtgta 1560
   acttaataag cctattccat cacaatcatg atcgctggta aagtagctca gtggtgtggg 1620
   gaaacgttcc cctggatcat actccagaat tctgctctca gcaattgcag ttaagtaagt 1680
   tacactacag ttctcacaag agcctgtgag gggatgtcag gtgcatcatt acattgggtg 1740
   tctcttttcc tagatttatg cttttgggat acagacctat gtttacaata taataaatat 1800
   tattgctatc ttttaaagat ataataatag gatgtaaact tgaccacaac tactgttttt 1860
   ttgaaataca tgattcatgg tttacatgtg tcaaggtgaa atctgagttg gcttttacag 1920
   atagttgact ttctatcttt tggcattctt tggtgtgtag aattactgta atacttctgc 1980
   aatcaactga aaactagagc ctttaaatga tttcaattcc acagaaagaa agtgagcttg 2040
   aacataggat gagctttaga aagaaaattg atcaagcaga tgtttaattg gaattgatta 2100
   ttagatccta ctttgtggat ttagtccctg ggattcagtc tgtagaaatg tctaatagtt 2160
   ctctatagtc cttgttcctg gtgaaccaca gttagggtgt tttgtttatt ttattgttct 2220
   tgctattgtt gatattctat gtagttgagc tctgtaaaag gaaattgtat tttatgtttt 2280
   agtaattgtt gccaactttt taaattaatt ttcattattt ttgagccaaa ttgaaatgtg 2340
   cacctcctgt gccttttttc tccttagaaa atctaattac ttggaacaag ttcagatttc 2400
   actggtcagt cattttcatc ttgttttctt cttgctaagt cttaccatgt acctgctttg 2460
   gcaatcattg caactctgag attataaaat gccttagaga atatactaac taataagatc 2520
   tttttttcag aaacagaaaa tagttccttg agtacttcct tcttgcattt ctgcctatgt 2580
   ttttgaagtt gttgctgttt gcctgcaata ggctataagg aatagcagga gaaattttac 2640
   tgaagtgctg ttttcctagg tgctactttg gcagagctaa gttatctttt gttttcttaa 2700
   tgcgtttgga ccattttgct ggctataaaa taactgatta atataattct aacacaatgt 2760
   tgacattgta gttacacaaa cacaaataaa tattttattt aaaattctgg aagtaatata 2820
   aaagggaaaa tatatttata agaaagggat aaaggtaata gagcccttct gccccccacc 2880
   caccaaattt acacaacaaa atgacatgtt cgaatgtgaa aggtcataat agctttccca 2940
   tcatgaatca gaaagatgtg gacagcttga tgttttagac aaccactgaa ctagatgact 3000
   gttgtactgt agctcagtca tttaaaaaat atataaatac taccttgtag tgtcccatac 3060
   tgtgtttttt acatggtaga ttcttattta agtgctaact ggttattttc tttggctggt 3120
   ttattgtact gttatacaga atgtaagttg tacagtgaaa taagttatta aagcatgtgt      3180
   aaacattgtt atatatcttt tctcctaaat ggagaatttt gaataaaata tatttgaaat      3240
   tttaaaaaaa aaaaaaaaaa a                                                3261
<210> 2
   <211> 30001
   <212> DNA
   <213> Homo sapiens
<400> 2
   caaagaaaag ggggaggttt tgttaaaaaa gagaaatgtt acatagtgct ctttgagaaa        60
   attcattggc actattaagg atctgaggag ctggtgagtt tcaactggtg agtgatggtg       120
   gtagataaaa ttagagctgc agcaggtcat tttagcaact attagataaa actggtctca       180
   ggtcacaacg ggcagttgca gcagctggac ttggagagaa ttacactgtg ggagcagtgt       240
   catttgtcct aagtgctttt ctacccccta cccccactat tttagttggg tataaaaaga       300
   atgacccaat ttgtatgatc aactttcaca aagcatagaa cagtaggaaa agggtctgtt       360
   tctgcagaag gtgtagacgt tgagagccat tttgtgtatt tattcctccc tttcttcctc       420
   ggtgaatgat taaaacgttc tgtgtgattt ttagtgatga aaaagattaa atgctactca       480
   ctgtagtaag tgccatctca cacttgcaga tcaaaaggca cacagtttaa aaaacctttg       540
   tttttttaca catctgagtg gtgtaaatgc tactcatctg tagtaagtgg aatctataca       600
   cctgcagacc aaaagacgca aggtttcaaa aatctttgtg ttttttacac atcaaacaga       660
   atggtacgtt tttcaaaagt taaaaaaaaa caactcatcc acatattgca actagcaaaa       720
   atgacattcc ccagtgtgaa aatcatgctt gagagaattc ttacatgtaa aggcaaaatt       780
   gcgatgactt tgcaggggac cgtgggattc ccgcccgcag tgccggagct gtcccctacc       840
   agggtttgca gtggagtttt gaatgcactt aacagtgtct tacggtaaaa acaaaatttc       900
   atccaccaat tatgtgttga gcgcccactg cctaccaagc acaaacaaaa ccattcaaaa       960
   ccacgaaatc gtcttcactt tctccagatc cagcagcctc ccctattaag gttcgcacac      1020
   gctattgcgc caacgctcct ccagagcggg tcttaagata aaagaacagg acaagttgcc      1080
   ccgccccatt tcgctagcct cgtgagaaaa cgtcatcgca catagaaaac agacagacgt      1140
   aacctacggt gtcccgctag gaaagagagg tgcgtcaaac agcgacaagt tccgcccacg      1200
   taaaagatga cgcttggtgt gtcagccgtc cctgctgccc ggttgcttct cttttggggg      1260
   cggggtctag caagagcagg tgtgggttta ggaggtgtgt gtttttgttt ttcccaccct      1320
   ctctccccac tacttgctct cacagtactc gctgagggtg aacaagaaaa gacctgataa      1380
   agattaacca gaagaaaaca aggagggaaa caaccgcagc ctgtagcaag ctctggaact      1440
   caggagtcgc gcgctagggg ccggggccgg ggccggggcg tggtcggggc gggcccgggg      1500
   gcgggcccgg ggcggggctg cggttgcggt gcctgcgccc gcggcggcgg aggcgcaggc 1560
   ggtggcgagt gggtgagtga ggaggcggca tcctggcggg tggctgtttg gggttcggct 1620
   gccgggaaga ggcgcgggta gaagcggggg ctctcctcag agctcgacgc atttttactt 1680
   tccctctcat ttctctgacc gaagctgggt gtcgggcttt cgcctctagc gactggtgga 1740
   attgcctgca tccgggcccc gggcttcccg gcggcggcgg cggcggcggc ggcgcaggga 1800
   caagggatgg ggatctggcc tcttccttgc tttcccgccc tcagtacccg agctgtctcc 1860
   ttcccgggga cccgctggga gcgctgccgc tgcgggctcg agaaaaggga gcctcgggta 1920
   ctgagaggcc tcgcctgggg gaaggccgga gggtgggcgg cgcgcggctt ctgcggacca 1980
   agtcggggtt cgctaggaac ccgagacggt ccctgccggc gaggagatca tgcgggatga 2040
   gatgggggtg tggagacgcc tgcacaattt cagcccaagc ttctagagag tggtgatgac 2100
   ttgcatatga gggcagcaat gcaagtcggt gtgctcccca ttctgtggga catgacctgg 2160
   ttgcttcaca gctccgagat gacacagact tgcttaaagg aagtgactat tgtgacttgg 2220
   gcatcacttg actgatggta atcagttgtc taaagaagtg cacagattac atgtccgtgt 2280
   gctcattggg tctatctggc cgcgttgaac accaccaggc tttgtattca gaaacaggag 2340
   ggaggtcctg cactttccca ggaggggtgg ccctttcaga tgcaatcgag attgttaggc 2400
   tctgggagag tagttgcctg gttgtggcag ttggtaaatt tctattcaaa cagttgccat 2460
   gcaccagttg ttcacaacaa gggtacgtaa tctgtctggc attacttcta cttttgtaca 2520
   aaggatcaaa aaaaaaaaag atactgttaa gatatgattt ttctcagact ttgggaaact 2580
   tttaacataa tctgtgaata tcacagaaac aagactatca tataggggat attaataacc 2640
   tggagtcaga atacttgaaa tacggtgtca tttgacacgg gcattgttgt caccacctct 2700
   gccaaggcct gccactttag gaaaaccctg aatcagttgg aaactgctac atgctgatag 2760
   tacatctgaa acaagaacga gagtaattac cacattccag attgttcact aagccagcat 2820
   ttacctgctc caggaaaaaa ttacaagcac cttatgaagt tgataaaata ttttgtttgg 2880
   ctatgttggc actccacaat ttgctttcag agaaacaaag taaaccaagg aggacttctg 2940
   tttttcaagt ctgccctcgg gttctattct acgttaatta gatagttccc aggaggacta 3000
   ggttagccta cctattgtct gagaaacttg gaactgtgag aaatggccag atagtgatat 3060
   gaacttcacc ttccagtctt ccctgatgtt gaagattgag aaagtgttgt gaactttctg 3120
   gtactgtaaa cagttcactg tccttgaagt ggtcctgggc agctcctgtt gtggaaagtg 3180
   gacggtttag gatcctgctt ctctttgggc tgggagaaaa taaacagcat ggttacaagt 3240
   attgagagcc aggttggaga aggtggctta cacctgtaat gccagagctt tgggaggcgg 3300
   aggcaagagg atcacttgaa gccaggagtt caagctcaac ctgggcaacg tagaccctgt 3360
   ctctacaaaa aattaaaaac ttagccgggc gtggtgatgt gcacctgtag tcctagctac 3420
   ttgggaggct gaggcaggag ggtcatttga gcccaagagt ttgaagttac cgagagctat 3480
   gatcctgcca gtgcattcca gcctggatga caaaacgaga ccctgtctct aaaaaacaag 3540
   aagtgagggc tttatgattg tagaattttc actacaatag cagtggacca accacctttc 3600
   taaataccaa tcagggaaga gatggttgat tttttaacag acgtttaaag aaaaagcaaa 3660
   acctcaaact tagcactcta ctaacagttt tagcagatgt taattaatgt aatcatgtct 3720
   gcatgtatgg gattatttcc agaaagtgta ttgggaaacc tctcatgaac cctgtgagca 3780
   agccaccgtc tcactcaatt tgaatcttgg cttccctcaa aagactggct aatgtttggt 3840
   aactctctgg agtagacagc actacatgta cgtaagatag gtacataaac aactattggt 3900
   tttgagctga tttttttcag ctgcatttgc atgtatggat ttttctcacc aaagacgatg 3960
   acttcaagta ttagtaaaat aattgtacag ctctcctgat tatacttctc tgtgacattt 4020
   catttcccag gctatttctt ttggtaggat ttaaaactaa gcaattcagt atgatctttg 4080
   tccttcattt tctttcttat tctttttgtt tgtttgtttg tttgtttttt tcttgaggca 4140
   gagtctctct ctgtcgccca ggctggagtg cagtggcgcc atctcagctc attgcaacct 4200
   ctgccacctc cgggttcaag agattctcct gcctcagcct cccgagtagc tgggattaca 4260
   ggtgtccacc accacacccg gctaattttt tgtattttta gtagaggtgg ggtttcacca 4320
   tgttggccag gctggtcttg agctcctgac ctcaggtgat ccacctgcct cggcctacca 4380
   aagagctggg ataacaggtg tgacccacca tgcccggccc attttttttt tcttattctg 4440
   ttaggagtga gagtgtaact agcagtataa tagttcaatt ttcacaacgt ggtaaaagtt 4500
   tccctataat tcaatcagat tttgctccag ggttcagttc tgttttagga aatactttta 4560
   ttttcagttt aatgatgaaa tattagagtt gtaatattgc ctttatgatt atccaccttt 4620
   ttaacctaaa agaatgaaag aaaaatatgt ttgcaatata attttatggt tgtatgttaa 4680
   cttaattcat tatgttggcc tccagtttgc tgttgttagt tatgacagca gtagtgtcat 4740
   taccatttca attcagatta cattcctata tttgatcatt gtaaactgac tgcttacatt 4800
   gtattaaaaa cagtggatat tttaaagaag ctgtacggct tatatctagt gctgtctctt 4860
   aagactatta aattgataca acatatttaa aagtaaatat tacctaaatg aatttttgaa 4920
   attacaaata cacgtgttaa aactgtcgtt gtgttcaacc atttctgtac atacttagag 4980
   ttaactgttt tgccaggctc tgtatgccta ctcataatat gataaaagca ctcatctaat 5040
   gctctgtaaa tagaagtcag tgctttccat cagactgaac tctcttgaca agatgtggat 5100
   gaaattcttt aagtaaaatt gtttactttg tcatacattt acagatcaaa tgttagctcc 5160
   caaagcaatc atatggcaaa gataggtata tcatagtttg cctattagct gctttgtatt 5220
   gctattatta taaatagact tcacagtttt agacttgctt aggtgaaatt gcaattcttt 5280
   ttactttcag tcttagataa caagtcttca attatagtac aatcacacat tgcttaggaa 5340
   tgcatcatta ggcgattttg tcattatgca aacatcatag agtgtactta cacaaaccta 5400
   gatagtatag cctttatgta cctaggccgt atggtatagt ctgttgctcc taggccacaa 5460
   acctgtacaa ctgttactgt actgaatact atagacagtt gtaacacagt ggtaaatatt 5520
   tatctaaata tatgcaaaca gagaaaaggt acagtaaaag tatggtataa aagataatgg 5580
   tatacctgtg taggccactt accacgaatg gagcttgcag gactagaagt tgctctgggt 5640
   gagtcagtga gtgagtggtg aattaatgtg aaggcctaga acactgtaca ccactgtaga 5700
   ctataaacac agtacgctga agctacacca aatttatctt aacagttttt cttcaataaa 5760
   aaattataac tttttaactt tgtaaacttt ttaatttttt aacttttaaa atacttagct 5820
   tgaaacacaa atacattgta tagctataca aaaatatttt ttctttgtat ccttattcta 5880
   gaagcttttt tctattttct attttaaatt ttttttttta cttgttagtc gtttttgtta 5940
   aaaactaaaa cacacacact ttcacctagg catagacagg attaggatca tcagtatcac 6000
   tcccttccac ctcactgcct tccacctcca catcttgtcc cactggaagg tttttagggg 6060
   caataacaca catgtagctg tcacctatga taacagtgct ttctgttgaa tacctcctga 6120
   aggacttgcc tgaggctgtt ttacatttaa cttaaaaaaa aaaaaagtag aaggagtgca 6180
   ctctaaaata acaataaaag gcatagtata gtgaatacat aaaccagcaa tgtagtagtt 6240
   tattatcaag tgttgtacac tgtaataatt gtatgtgcta tactttaaat aacttgcaaa 6300
   atagtactaa gaccttatga tggttacagt gtcactaagg caatagcata ttttcaggtc 6360
   cattgtaatc taatgggact accatcatat atgcagtcta ccattgactg aaacgttaca 6420
   tggcacataa ctgtatttgc aagaatgatt tgttttacat taatatcaca taggatgtac 6480
   ctttttagag tggtatgttt atgtggatta agatgtacaa gttgagcaag gggaccaaga 6540
   gccctgggtt ctgtcttgga tgtgagcgtt tatgttcttc tcctcatgtc tgttttctca 6600
   ttaaattcaa aggcttgaac gggccctatt tagcccttct gttttctacg tgttctaaat 6660
   aactaaagct tttaaattct agccatttag tgtagaactc tctttgcagt gatgaaatgc 6720
   tgtattggtt tcttggctag catattaaat atttttatct ttgtcttgat acttcaatgt 6780
   cgttttaaac atcaggatcg ggcttcagta ttctcataac cagagagttc actgaggata 6840
   caggactgtt tgcccatttt ttgttatggc tccagacttg tggtatttcc atgtcttttt 6900
   tttttttttt ttttttgacc ttttagcggc tttaaagtat ttctgttgtt aggtgttgta 6960
   ttacttttct aagattactt aacaaagcac cacaaactga gtggctttaa acaacagcaa 7020
   tttattctct cacaattcta gaagctagaa gtccgaaatc aaagtgttga caggggcatg 7080
   atcttcaaga gagaagactc tttccttgcc tcttcctggc ttctggtggt taccagcaat 7140
   cctgagtgtt cctttcttgc cttgtagttt caacaatcca gtatctgcct tttgtcttca 7200
   catggctgtc taccatttgt ctctgtgtct ccaaatctct ctccttataa acacagcagt 7260
   tattggatta ggccccactc taatccagta tgaccccatt ttaacatgat tacacttatt 7320
   tctagataag gtcacattca cgtacaccaa gggttaggaa ttgaacatat ctttttgggg 7380
   gacacaattc aacccacaag tgtcagtctc tagctgagcc tttcccttcc tgtttttctc 7440
   ctttttagtt gctatgggtt aggggccaaa tctccagtca tactagaatt gcacatggac 7500
   tggatatttg ggaatactgc gggtctattc tatgagcttt agtatgtaac atttaatatc 7560
   agtgtaaaga agcccttttt taagttattt ctttgaattt ctaaatgtat gccctgaata 7620
   taagtaacaa gttaccatgt cttgtaaaat gatcatatca acaaacattt aatgtgcacc 7680
   tactgtgcta gttgaatgtc tttatcctga taggagataa caggattcca catctttgac 7740
   ttaagaggac aaaccaaata tgtctaaatc atttggggtt ttgatggata tctttaaatt 7800
   gctgaaccta atcattggtt tcatatgtca ttgtttagat atctccggag catttggata 7860
   atgtgacagt tggaatgcag tgatgtcgac tctttgccca ccgccatctc cagctgttgc 7920
   caagacagag attgctttaa gtggcaaatc acctttatta gcagctactt ttgcttactg 7980
   ggacaatatt cttggtccta gagtaaggca catttgggct ccaaagacag aacaggtact 8040
   tctcagtgat ggagaaataa cttttcttgc caaccacact ctaaatggag aaatccttcg 8100
   aaatgcagag agtggtgcta tagatgtaaa gttttttgtc ttgtctgaaa agggagtgat 8160
   tattgtttca ttaatctttg atggaaactg gaatggggat cgcagcacat atggactatc 8220
   aattatactt ccacagacag aacttagttt ctacctccca cttcatagag tgtgtgttga 8280
   tagattaaca catataatcc ggaaaggaag aatatggatg cataaggtaa gtgatttttc 8340
   agcttattaa tcatgttaac ctatctgttg aaagcttatt ttctggtaca tataaatctt 8400
   atttttttaa ttatatgcag tgaacatcaa acaataaatg ttatttattt tgcatttacc 8460
   ctattagata caaatacatc tggtctgata cctgtcatct tcatattaac tgtggaaggt 8520
   acgaaatggt agctccacat tatagatgaa aagctaaagc ttagacaaat aaagaaactt 8580
   ttagaccctg gattcttctt gggagccttt gactctaata ccttttgttt ccctttcatt 8640
   gcacaattct gtcttttgct tactactatg tgtaagtata acagttcaaa gtaatagttt 8700
   cataagctgt tggtcatgta gcctttggtc tctttaacct ctttgccaag ttcccaggtt 8760
   cataaaatga ggaggttgaa tggaatggtt cccaagagaa ttccttttaa tcttacagaa 8820
   attattgttt tcctaaatcc tgtagttgaa tatataatgc tatttacatt tcagtatagt 8880
   tttgatgtat ctaaagaaca cattgaattc tccttcctgt gttccagttt gatactaacc 8940
   tgaaagtcca ttaagcatta ccagttttaa aaggcttttg cccaatagta aggaaaaata 9000
   atatctttta aaagaataat tttttactat gtttgcaggc ttacttcctt ttttctcaca 9060
   ttatgaaact cttaaaatca ggagaatctt ttaaacaaca tcataatgtt taatttgaaa 9120
   agtgcaagtc attcttttcc tttttgaaac tatgcagatg ttacattgac tgttttctgt 9180
   gaagttatct ttttttcact gcagaataaa ggttgttttg attttatttt gtattgttta 9240
   tgagaacatg catttgttgg gttaatttcc tacccctgcc cccatttttt ccctaaagta 9300
   gaaagtattt ttcttgtgaa ctaaattact acacaagaac atgtctattg aaaaataagc 9360
   aagtatcaaa atgttgtggg ttgttttttt aaataaattt tctcttgctc aggaaagaca 9420
   agaaaatgtc cagaagatta tcttagaagg cacagagaga atggaagatc aggtatatgc 9480
   aaattgcata ctgtcaaatg tttttctcac agcatgtatc tgtataaggt tgatggctac 9540
   atttgtcaag gccttggaga catacgaata agcctttaat ggagctttta tggaggtgta 9600
   cagaataaac tggaggaaga tttccatatc ttaaacccaa agagttaaat cagtaaacaa 9660
   aggaaaatag taattgcatc tacaaattaa tatttgctcc cttttttttt ctgtttgccc 9720
   agaataaatt ttggataact tgttcatagt aaaaataaaa aaaattgtct ctgatatgtt 9780
   ctttaaggta ctacttctcg aacctttccc tagaagtagc tgtaacagaa ggagagcata 9840
   tgtacccctg aggtatctgt ctggggtgta ggcccaggtc cacacaatat ttcttctaag 9900
   tcttatgttg tatcgttaag actcatgcaa tttacatttt attccataac tattttagta 9960
   ttaaaatttg tcagtgatat ttcttaccct ctcctctagg aaaatgtgcc atgtttatcc 10020
   cttggctttg aatgcccctc aggaacagac actaagagtt tgagaagcat ggttacaagg 10080
   gtgtggcttc ccctgcggaa actaagtaca gactatttca ctgtaaagca gagaagttct 10140
   tttgaaggag aatctccagt gaagaaagag ttcttcactt ttacttccat ttcctcttgt 10200
   gggtgaccct caatgctcct tgtaaaactc caatatttta aacatggctg ttttgccttt 10260
   ctttgcttct ttttagcatg aatgagacag atgatacttt aaaaaagtaa ttaaaaaaaa 10320
   aaacttgtga aaatacatgg ccataataca gaacccaata caatgatctc ctttaccaaa 10380
   ttgttatgtt tgtacttttg tagatagctt tccaattcag agacagttat tctgtgtaaa 10440
   ggtctgactt aacaagaaaa gatttccctt tacccaaaga atcccagtcc ttatttgctg 10500
   gtcaataagc agggtcccca ggaatggggt aactttcagc accctctaac ccactagtta 10560
   ttagtagact aattaagtaa acttatcgca agttgaggaa acttagaacc aactaaaatt 10620
   ctgcttttac tgggattttg ttttttcaaa ccagaaacct ttacttaagt tgactactat 10680
   taatgaattt tggtctctct tttaagtgct cttcttaaaa atgttatctt actgctgaga 10740
   agttcaagtt tgggaagtac aaggaggaat agaaacttaa gagattttct tttagagcct 10800
   cttctgtatt tagccctgta ggattttttt tttttttttt ttttttggtg ttgttgagct 10860
   tcagtgaggc tattcattca cttatactga taatgtctga gatactgtga atgaaatact 10920
   atgtatgctt aaacctaaga ggaaatattt tcccaaaatt attcttcccg aaaaggagga 10980
   gttgcctttt gattgagttc ttgcaaatct cacaacgact ttattttgaa caatactgtt 11040
   tggggatgat gcattagttt gaaacaactt cagttgtagc tgtcatctga taaaattgct 11100
   tcacagggaa ggaaatttaa cacggatcta gtcattattc ttgttagatt gaatgtgtga 11160
   attgtaattg taaacaggca tgataattat tactttaaaa actaaaaaca gtgaatagtt 11220
   agttgtggag gttactaaag gatggttttt ttttaaataa aactttcagc attatgcaaa 11280
   tgggcatatg gcttaggata aaacttccag aagtagcatc acatttaaat tctcaagcaa 11340
   cttaataata tggggctctg aaaaactggt taaggttact ccaaaaatgg ccctgggtct 11400
   gacaaagatt ctaacttaaa gatgcttatg aagactttga gtaaaatcat ttcataaaat 11460
   aagtgaggaa aaacaactag tattaaattc atcttaaata atgtatgatt taaaaaatat 11520
   gtttagctaa aaatgcatag tcatttgaca atttcattta tatctcaaaa aatttactta 11580
   accaagttgg tcacaaaact gatgagactg gtggtggtag tgaataaatg agggaccatc 11640
   catatttgag acactttaca tttgtgatgt gttatactga attttcagtt tgattctata 11700
   gactacaaat ttcaaaatta caatttcaag atgtaataag tagtaatatc ttgaaatagc 11760
   tctaaaggga atttttctgt tttattgatt cttaaaatat atgtgctgat tttgatttgc 11820
   atttgggtag attatacttt tatgagtatg gaggttaggt attgattcaa gttttcctta 11880
   cctatttggt aaggatttca aagtcttttt gtgcttggtt ttcctcattt ttaaatatga 11940
   aatatattga tgacctttaa caaatttttt ttatctcaaa ttttaaagga gatcttttct 12000
   aaaagaggca tgatgactta atcattgcat gtaacagtaa acgataaacc aatgattcca 12060
   tactctctaa agaataaaag tgagctttag ggccgggcat ggtcagaaat ttgacaccaa 12120
   cctggccaac atggcgaaac cccgtctcta ctaaaaatac aaaaatcagc cgggcatggt 12180
   ggcggcacct atagtcccag ctacttggga ggatgagaca ggagagtcac ttgaacctgg 12240
   gaggagaggt tgcagtgagc tgagatcacg ccattgcact ccagcctgag caatgaaagc 12300
   aaaactccat ctcaaaaaaa aaaaaagaaa agaaagaata aaagtgagct ttggattgca 12360
   tataaatcct ttagacatgt agtagacttg tttgatactg tgtttgaaca aattacgaag 12420
   tattttcatc aaagaatgtt attgtttgat gttattttta ttttttattg cccagcttct 12480
   ctcatattac gtgattttct tcacttcatg tcactttatt gtgcagggtc agagtattat 12540
   tccaatgctt actggagaag tgattcctgt aatggaactg ctttcatcta tgaaatcaca 12600
   cagtgttcct gaagaaatag atgtaagttt aaatgagagc aattatacac tttatgagtt 12660
   ttttggggtt atagtattat tatgtatatt attaatattc taattttaat agtaaggact 12720
   ttgtcataca tactattcac atacagtatt agccacttta gcaaataagc acacacaaaa 12780
   tcctggattt tatggcaaaa cagaggcatt tttgatcagt gatgacaaaa ttaaattcat 12840
   tttgtttatt tcattacttt tataattcct aaaagtggga ggatcccagc tcttatagga 12900
   gcaattaata tttaatgtag tgtcttttga aacaaaactg tgtgccaaag tagtaaccat 12960
   taatggaagt ttacttgtag tcacaaattt agtttcctta atcatttgtt gaggacgttt 13020
   tgaatcacac actatgagtg ttaagagata cctttaggaa actattcttg ttgttttctg 13080
   attttgtcat ttaggttagt ctcctgattc tgacagctca gaagaggaag ttgttcttgt 13140
   aaaaattgtt taacctgctt gaccagcttt cacatttgtt cttctgaagt ttatggtagt 13200
   gcacagagat tgttttttgg ggagtcttga ttctcggaaa tgaaggcagt gtgttatatt 13260
   gaatccagac ttccgaaaac ttgtatatta aaagtgttat ttcaacacta tgttacagcc 13320
   agactaattt ttttattttt tgatgcattt tagatagctg atacagtact caatgatgat 13380
   gatattggtg acagctgtca tgaaggcttt cttctcaagt aagaattttt cttttcataa 13440
   aagctggatg aagcagatac catcttatgc tcacctatga caagatttgg aagaaagaaa 13500
   ataacagact gtctacttag attgttctag ggacattacg tatttgaact gttgcttaaa 13560
   tttgtgttat ttttcactca ttatatttct atatatattt ggtgttattc catttgctat 13620
   ttaaagaaac cgagtttcca tcccagacaa gaaatcatgg ccccttgctt gattctggtt 13680
   tcttgtttta cttctcatta aagctaacag aatcctttca tattaagttg tactgtagat 13740
   gaacttaagt tatttaggcg tagaacaaaa ttattcatat ttatactgat ctttttccat 13800
   ccagcagtgg agtttagtac ttaagagttt gtgcccttaa accagactcc ctggattaat 13860
   gctgtgtacc cgtgggcaag gtgcctgaat tctctataca cctatttcct catctgtaaa 13920
   atggcaataa tagtaatagt acctaatgtg tagggttgtt ataagcattg agtaagataa 13980
   ataatataaa gcacttagaa cagtgcctgg aacataaaaa cacttaataa tagctcatag 14040
   ctaacatttc ctatttacat ttcttctaga aatagccagt atttgttgag tgcctacatg 14100
   ttagttcctt tactagttgc tttacatgta ttatcttata ttctgtttta aagtttcttc 14160
   acagttacag attttcatga aattttactt ttaataaaag agaagtaaaa gtataaagta 14220
   ttcactttta tgttcacagt cttttccttt aggctcatga tggagtatca gaggcatgag 14280
   tgtgtttaac ctaagagcct taatggcttg aatcagaagc actttagtcc tgtatctgtt 14340
   cagtgtcagc ctttcataca tcattttaaa tcccatttga ctttaagtaa gtcacttaat 14400
   ctctctacat gtcaatttct tcagctataa aatgatggta tttcaataaa taaatacatt 14460
   aattaaatga tattatactg actaattggg ctgttttaag gctcaataag aaaatttctg 14520
   tgaaaggtct ctagaaaatg taggttccta tacaaataaa agataacatt gtgcttatag 14580
   cttcggtgtt tatcatataa agctattctg agttatttga agagctcacc tacttttttt 14640
   tgtttttagt ttgttaaatt gttttatagg caatgttttt aatctgtttt ctttaactta 14700
   cagtgccatc agctcacact tgcaaacctg tggctgttcc gttgtagtag gtagcagtgc 14760
   agagaaagta aataaggtag tttattttat aatctagcaa atgatttgac tctttaagac    14820
   tgatgatata tcatggattg tcatttaaat ggtaggttgc aattaaaatg atctagtagt    14880
   ataaggaggc aatgtaatct catcaaattg ctaagacacc ttgtggcaac agtgagtttg    14940
   aaataaactg agtaagaatc atttatcagt ttattttgat agctcggaaa taccagtgtc    15000
   agtagtgtat aaatggtttt gagaatatat taaaatcaga tatataaaaa aaattactct    15060
   tctatttccc aatgttatct ttaacaaatc tgaagatagt catgtacttt tggtagtagt    15120
   tccaaagaaa tgttatttgt ttattcatct tgatttcatt gtcttcgctt tccttctaaa    15180
   tctgtccctt ctagggagct attgggatta agtggtcatt gattattata ctttattcag    15240
   taatgtttct gaccctttcc ttcagtgcta cttgagttaa ttaaggatta atgaacagtt    15300
   acatttccaa gcattagcta ataaactaaa ggattttgca cttttcttca ctgaccatta    15360
   gttagaaaga gttcagagat aagtatgtgt atctttcaat ttcagcaaac ctaatttttt    15420
   aaaaaaagtt ttacatagga aatatgttgg aaatgatact ttacaaagat attcataatt    15480
   tttttttgta atcagctact ttgtatattt acatgagcct taatttatat ttctcatata    15540
   accatttatg agagcttagt atacctgtgt cattatattg catctacgaa ctagtgacct    15600
   tattccttct gttacctcaa acaggtggct ttccatctgt gatctccaaa gccttaggtt    15660
   gcacagagtg actgccgagc tgctttatga agggagaaag gctccatagt tggagtgttt    15720
   tttttttttt ttttaaacat ttttcccatc ctccatcctc ttgagggaga atagcttacc    15780
   ttttatcttg ttttaatttg agaaagaagt tgccaccact ctaggttgaa aaccactcct    15840
   ttaacataat aactgtggat atggtttgaa tttcaagata gttacatgcc tttttatttt    15900
   tcctaataga gctgtaggtc aaatattatt agaatcagat ttctaaatcc cacccaatga    15960
   cctgcttatt ttaaatcaaa ttcaataatt aattctcttc tttttggagg atctggacat    16020
   tctttgatat ttcttacaac gaatttcatg tgtagaccca ctaaacagaa gctataaaag    16080
   ttgcatggtc aaataagtct gagaaagtct gcagatgata taattcacct gaagagtcac    16140
   agtatgtagc caaatgttaa aggttttgag atgccataca gtaaatttac caagcatttt    16200
   ctaaatttat ttgaccacag aatccctatt ttaagcaaca actgttacat cccatggatt    16260
   ccaggtgact aaagaatact tatttcttag gatatgtttt attgataata acaattaaaa    16320
   tttcagatat ctttcataag caaatcagtg gtctttttac ttcatgtttt aatgctaaaa    16380
   tattttcttt tatagatagt cagaacatta tgcctttttc tgactccagc agagagaaaa    16440
   tgctccaggt tatgtgaagc agaatcatca tttaaatatg agtcagggct ctttgtacaa    16500
   ggcctgctaa aggtatagtt tctagttatc acaagtgaaa ccacttttct aaaatcattt    16560
   ttgagactct ttatagacaa atcttaaata ttagcattta atgtatctca tattgacatg    16620
   cccagagact gacttccttt acacagttct gcacatagac tatatgtctt atggatttat 16680
   agttagtatc atcagtgaaa caccatagaa taccctttgt gttccaggtg ggtccctgtt 16740
   cctacatgtc tagcctcagg actttttttt ttttaacaca tgcttaaatc aggttgcaca 16800
   tcaaaaataa gatcatttct ttttaactaa atagatttga attttattga aaaaaaattt 16860
   taaacatctt taagaagctt ataggattta agcaattcct atgtatgtgt actaaaatat 16920
   atatatttct atatataata tatattagaa aaaaattgta tttttctttt atttgagtct 16980
   actgtcaagg agcaaaacag agaaatgtaa attagcaatt atttataata cttaaaggga 17040
   agaaagttgt tcaccttgtt gaatctatta ttgttatttc aattatagtc ccaagacgtg 17100
   aagaaatagc tttcctaatg gttatgtgat tgtctcatag tgactacttt cttgaggatg 17160
   tagccacggc aaaatgaaat aaaaaaattt aaaaattgtt gcaaatacaa gttatattag 17220
   gcttttgtgc attttcaata atgtgctgct atgaactcag aatgatagta tttaaatata 17280
   gaaactagtt aaaggaaacg tagtttctat ttgagttata catatctgta aattagaact 17340
   tctcctgtta aaggcataat aaagtgctta atacttttgt ttcctcagca ccctctcatt 17400
   taattatata attttagttc tgaaagggac ctataccaga tgcctagagg aaatttcaaa 17460
   actatgatct aatgaaaaaa tatttaatag ttctccatgc aaatacaaat catatagttt 17520
   tccagaaaat acctttgaca ttatacaaag atgattatca cagcattata atagtaaaaa 17580
   aatggaaata gcctctttct tctgttctgt tcatagcaca gtgcctcata cgcagtaggt 17640
   tattattaca tggtaactgg ctaccccaac tgattaggaa agaagtaaat ttgttttata 17700
   aaaatacata ctcattgagg tgcatagaat aattaagaaa ttaaaagaca cttgtaattt 17760
   tgaatccagt gaatacccac tgttaatatt tggtatatct ctttctagtc tttttttccc 17820
   ttttgcatgt attttcttta agactcccac ccccactgga tcatctctgc atgttctaat 17880
   ctgctttttt cacagcagat tctaagcctc tttgaatatc aacacaaact tcaacaactt 17940
   catctataga tgccaaataa taaattcatt tttatttact taaccacttc ctttggatgc 18000
   ttaggtcatt ctgatgtttt gctattgaaa ccaatgctat actgaacact tctgtcacta 18060
   aaactttgca cacactcatg aatagcttct taggataaat ttttagagat ggatttgcta 18120
   aatcagagac cattttttaa aattaaaaaa caattattca tatcgtttgg catgtaagac 18180
   agtaaatttt ccttttattt tgacaggatt caactggaag ctttgtgctg cctttccggc 18240
   aagtcatgta tgctccatat cccaccacac acatagatgt ggatgtcaat actgtgaagc 18300
   agatgccacc ctgtcatgaa catatttata atcagcgtag atacatgaga tccgagctga 18360
   cagccttctg gagagccact tcagaagaag acatggctca ggatacgatc atctacactg 18420
   acgaaagctt tactcctgat ttgtacgtaa tgctctgcct gctggtactg tagtcaagca 18480
   atatgaaatt gtgtctttta cgaataaaaa caaaacagaa gttgcattta aaaagaaaga 18540
   aatattacca gcagaattat gcttgaagaa acatttaatc aagcattttt ttcttaaatg 18600
   ttcttctttt tccatacaat tgtgtttacc ctaaaatagg taagattaac ccttaaagta 18660
   aatatttaac tatttgttta ataaatatat attgagctcc taggcactgt tctaggtacc 18720
   gggcttaata gtggccaacc agacagcccc agccccagcc cctacattgt gtatagtcta 18780
   ttatgtaaca gttattgaat ggacttatta acaaaaccaa agaagtaatt ctaagtcttt 18840
   tttttcttga catatgaata taaaatacag caaaactgtt aaaatatatt aatggaacat 18900
   ttttttactt tgcattttat attgttattc acttcttatt tttttttaaa aaaaaaagcc 18960
   tgaacagtaa attcaaaagg aaaagtaatg ataattaatt gttgagcatg gacccaactt 19020
   gaaaaaaaaa atgatgatga taaatctata atcctaaaac cctaagtaaa cacttaaaag 19080
   atgttctgaa atcaggaaaa gaattatagt atacttttgt gtttctcttt tatcagttga 19140
   aaaaaggcac agtagctcat gcctgtaaga acagagcttt gggagtgcaa ggcaggcgga 19200
   tcacttgagg ccaggagttc cagaccagcc tgggcaacat agtgaaaccc catctctaca 19260
   aaaaataaaa aagaattatt ggaatgtgtt tctgtgtgcc tgtaatccta gctattccga 19320
   aagctgaggc aggaggatct tttgagccca ggagtttgag gttacaggga gttatgatgt 19380
   gccagtgtac tccagcctgg ggaacaccga gactctgtct tatttaaaaa aaaaaaaaaa 19440
   aaaatgcttg caataatgcc tggcacatag aaggtaacag taagtgttaa ctgtaataac 19500
   ccaggtctaa gtgtgtaagg caatagaaaa attggggcaa ataagcctga cctatgtatc 19560
   tacagaatca gtttgagctt aggtaacaga cctgtggagc accagtaatt acacagtaag 19620
   tgttaaccaa aagcatagaa taggaatatc ttgttcaagg gacccccagc cttatacatc 19680
   tcaaggtgca gaaagatgac ttaatatagg acccattttt tcctagttct ccagagtttt 19740
   tattggttct tgagaaagta gtaggggaat gttttagaaa atgaattggt ccaactgaaa 19800
   ttacatgtca gtaagttttt atatattggt aaattttagt agacatgtag aagttttcta 19860
   attaatctgt gccttgaaac attttctttt ttcctaaagt gcttagtatt ttttccgttt 19920
   tttgattggt tacttgggag cttttttgag gaaatttagt gaactgcaga atgggtttgc 19980
   aaccatttgg tatttttgtt ttgtttttta gaggatgtat gtgtatttta acatttctta 20040
   atcattttta gccagctatg tttgttttgc tgatttgaca aactacagtt agacagctat 20100
   tctcattttg ctgatcatga caaaataata tcctgaattt ttaaattttg catccagctc 20160
   taaattttct aaacataaaa ttgtccaaaa aatagtattt tcagccacta gattgtgtgt 20220
   taagtctatt gtcacagagt cattttactt ttaagtatat gtttttacat gttaattatg 20280
   tttgttattt ttaattttaa ctttttaaaa taattccagt cactgccaat acatgaaaaa 20340
   ttggtcactg gaattttttt tttgactttt attttaggtt catgtgtaca tgtgcaggtg 20400
   tgttatacag gtaaattgcg tgtcatgagg gtttggtgta caggtgattt cattacccag 20460
   gtaataagca tagtacccaa taggtagttt tttgatcctc acccttctcc caccctcaag 20520
   taggccctgg tgttgctgtt tccttctttg tgtccatgta tactcagtgt ttagctccca 20580
   cttagaagtg agaacatgcg gtagttggtt ttctgttcct ggattagttc acttaggata 20640
   atgacctcta gctccatctg gtttttatgg ctgcatagta ttccatggtg tatatgtatc 20700
   acattttctt tatccagtct accattgata ggcatttagg ttgattccct gtctttgtta 20760
   tcatgaatag tgctgtgatg aacatacaca tgcatgtgtc tttatggtag aaaaatttgt 20820
   attcctttag gtacatatag aataatgggg ttgctagggt gaatggtagt tctattttca 20880
   gttatttgag aaatcttcaa actgcttttc ataatagcta aactaattta cagtcccgcc 20940
   agcagtgtat aagtgttccc ttttctccac aaccttgcca acatctgtga ttttttgact 21000
   ttttaataat agccattcct agagaattga tttgcaattc tctattagtg atattaagca 21060
   ttttttcata tgctttttag ctgtctgtat atattcttct gaaaaatttt catgtccttt 21120
   gcccagtttg tagtggggtg ggttgttttt tgcttgttaa ttagttttaa gttccttcca 21180
   gattctgcat atccctttgt tggatacatg gtttgcagat atttttctcc cattgtgtag 21240
   gttgtctttt actctgttga tagtttcttt tgccatgcag gagctcgtta ggtcccattt 21300
   gtgtttgttt ttgttgcagt tgcttttggc gtcttcatca taaaatctgt gccagggcct 21360
   atgtccagaa tggtatttcc taggttgtct tccagggttt ttacaatttt agattttacg 21420
   tttatgtctt taatccatct tgagttgatt tttgtatatg gcacaaggaa ggggtccagt 21480
   ttcactccaa ttcctatggc tagcaattat cccagcacca tttattgaat acggagtcct 21540
   ttccccattg cttgtttttt gtcaactttg ttgaagatca gatggttgta agtgtgtggc 21600
   tttatttctt ggctctctat tctccattgg tctatgtgtc tgtttttata acagtaccct 21660
   gctgttcagg ttcctatagc cttttagtat aaaatcggct aatgtgatgc ctccagcttt 21720
   gttctttttg cttaggattg ctttggctat ttgggctcct ttttgggtcc atattaattt 21780
   taaaacagtt ttttctggtt ttgtgaagga tatcattggt agtttatagg aatagcattg 21840
   aatctgtaga ttgctttggg cagtatggcc attttaacaa tattaattct tcctatctat 21900
   gaatatggaa tgtttttcca tgtgtttgtg tcatctcttt atacctgatg tataaagaaa 21960
   agctggtatt attcctactc aatctgttcc aaaaaattga ggaggaggaa ctcttcccta 22020
   atgaggccag catcattctg ataccaaaac ctggcagaga cacaacagaa aaaagaaaac 22080
   ttcaggccaa tatccttgat gaatatagat gcaaaaatcc tcaacaaaat actagcaaac 22140
   caaatccagc agcacatcaa aaagctgatc tactttgatc aagtaggctt tatccctggg 22200
   atgcaaggtt ggttcaacat acacaaatca ataagtgtga ttcatcacat aaacagagct 22260
   aaaaacaaaa accacaagat tatctcaata ggtagagaaa aggttgtcaa taaaatttaa 22320
   catcctccat gttaaaaacc ttcagtaggt caggtgtagt gactcacacc tgtaatccca 22380
   gcactttggg aggccaaggc gggcatatct cttaagccca ggagttcaag acgagcctag 22440
   gcagcatggt gaaaccccat ctctacaaaa aaaaaaaaaa aaaaaaatta gcttggtatg 22500
   gtgacatgca cctatagtcc cagctattca ggaggttgag gtgggaggat tgtttgagcc 22560
   cgggaggcag aggttggcag cgagctgaga tcatgccacc gcactccagc ctgggcaacg 22620
   gagtgagacc ctgtctcaaa aaagaaaaat cacaaacaat cctaaacaaa ctaggcattg 22680
   aaggaacatg cctcaaaaaa ataagaacca tctatgacag acccatagcc aatatcttac 22740
   caaatgggca aaagctggaa gtattctcct tgagaaccgt aacaagacaa ggatgtccac 22800
   tctcaccact ccttttcagc atagttctgg aagtcctagc cagagcaatc aggaaagaga 22860
   aagaaagaaa gacattcaga taggaagaga agaagtcaaa ctatttctgt ttgcaggcag 22920
   tataattctg tacctagaaa atctcatagt ctctgcccag aaactcctaa atctgttaaa 22980
   aatttcagca aagttttggc attctctata ctccaacacc ttccaaagtg agagcaaaat 23040
   caagaacaca gtcccattca caatagccgc aaaacgaata aaatacctag gaatccagct 23100
   aaccagggag gtgaaagatc tctatgagaa ttacaaaaca ctgctgaaag aaatcagaga 23160
   tgacacaaac aaatggaaat gttctttttt aacaccttgc tttatctaat tcacttatga 23220
   tgaagatact cattcagtgg aacaggtata ataagtccac tcgattaaat ataagcctta 23280
   ttctctttcc agagcccaag aaggggcact atcagtgccc agtcaataat gacgaaatgc 23340
   taatattttt cccctttacg gtttctttct tctgtagtgt ggtacactcg tttcttaaga 23400
   taaggaaact tgaactacct tcctgtttgc ttctacacat acccattctc tttttttgcc 23460
   actctggtca ggtataggat gatccctacc actttcagtt aaaaactcct cctcttacta 23520
   aatgttctct taccctctgg cctgagtaga acctagggaa aatggaagag aaaaagatga 23580
   aagggaggtg gggcctggga agggaataag tagtcctgtt tgtttgtgtg tttgctttag 23640
   cacctgctat atcctaggtg ctgtgttagg cacacattat tttaagtggc cattatatta 23700
   ctactactca ctctggtcgt tgccaaggta ggtagtactt tcttggatag ttggttcatg 23760
   ttacttacag atggtgggct tgttgaggca aacccagtgg ataatcatcg gagtgtgttc 23820
   tctaatctca ctcaaatttt tcttcacatt ttttggtttg ttttggtttt tgatggtagt 23880
   ggcttatttt tgttgctggt ttgttttttg tttttttttg agatggcaag aattggtagt 23940
   tttatttatt aattgcctaa gggtctctac tttttttaaa agatgagagt agtaaaatag 24000
   attgatagat acatacatac ccttactggg gactgcttat attctttaga gaaaaaatta 24060
   catattagcc tgacaaacac cagtaaaatg taaatatatc cttgagtaaa taaatgaatg 24120
   tatattttgt gtctccaaat atatatatct atattcttac aaatgtgttt atatgtaata 24180
   tcaatttata agaacttaaa atgttggctc aagtgaggga ttgtggaagg tagcattata 24240
   tggccatttc aacatttgaa cttttttctt ttcttcattt tcttcttttc ttcaggaata 24300
   tttttcaaga tgtcttacac agagacactc tagtgaaagc cttcctggat caggtaaatg 24360
   ttgaacttga gattgtcaga gtgaatgata tgacatgttt tcttttttaa tatatcctac 24420
   aatgcctgtt ctatatattt atattcccct ggatcatgcc ccagagttct gctcagcaat 24480
   tgcagttaag ttagttacac tacagttctc agaagagtct gtgagggcat gtcaagtgca 24540
   tcattacatt ggttgcctct tgtcctagat ttatgcttcg ggaattcaga cctttgttta 24600
   caatataata aatattattg ctatctttta aagatataat aataagatat aaagttgacc 24660
   acaactactg ttttttgaaa catagaattc ctggtttaca tgtatcaaag tgaaatctga 24720
   cttagctttt acagatataa tatatacata tatatatcct gcaatgcttg tactatatat 24780
   gtagtacaag tatatatata tgtttgtgtg tgtatatata tatagtacga gcatatatac 24840
   atattaccag cattgtagga tatatatatg tttatatatt aaaaaaaagt tataaactta 24900
   aaaccctatt atgttatgta gagtatatgt tatatatgat atgtaaaata tataacatat 24960
   actctatgat agagtgtaat atatttttta tatatatttt aacatttata aaatgataga 25020
   attaagaatt gagtcctaat ctgttttatt aggtgctttt tgtagtgtct ggtctttcta 25080
   aagtgtctaa atgatttttc cttttgactt attaatgggg aagagcctgt atattaacaa 25140
   ttaagagtgc agcattccat acgtcaaaca acaaacattt taattcaagc attaacctat 25200
   aacaagtaag tttttttttt ttttttgaga aagggaggtt gtttatttgc ctgaaatgac 25260
   tcaaaaatat ttttgaaaca tagtgtactt atttaaataa catctttatt gtttcattct 25320
   tttaaaaaat atctacttaa ttacacagtt gaaggaaatc gtagattata tggaacttat 25380
   ttcttaatat attacagttt gttataataa cattctgggg atcaggccag gaaactgtgt 25440
   catagataaa gctttgaaat aatgagatcc ttatgtttac tagaaatttt ggattgagat 25500
   ctatgaggtc tgtgacatat tgcgaagttc aaggaaaatt cgtaggcctg gaatttcatg 25560
   cttctcaagc tgacataaaa tccctcccac tctccacctc atcatatgca cacattctac 25620
   tcctacccac ccactccacc ccctgcaaaa gtacaggtat atgaatgtct caaaaccata 25680
   ggctcatctt ctaggagctt caatgttatt tgaagatttg ggcagaaaaa attaagtaat 25740
   acgaaataac ttatgtatga gttttaaaag tgaagtaaac atggatgtat tctgaagtag 25800
   aatgcaaaat ttgaatgcat ttttaaagat aaattagaaa acttctaaaa actgtcagat 25860
   tgtctgggcc tggtggctta tgcctgtaat cccagcactt tgggagtccg aggtgggtgg 25920
   atcacaaggt caggagatcg agaccatcct gccaacatgg tgaaaccccg tctctactaa 25980
   gtatacaaaa attagctggg cgtggcagcg tgtgcctgta atcccagcta cctgggaggc 26040
   tgaggcagga gaatcgcttg aacccaggag gtgtaggttg cagtgagtca agatcgcgcc 26100
   actgcacttt agcctggtga cagagctaga ctccgtctca aaaaaaaaaa aaaatatcag 26160
   attgttccta cacctagtgc ttctatacca cactcctgtt agggggcatc agtggaaatg 26220
   gttaaggaga tgtttagtgt gtattgtctg ccaagcactg tcaacactgt catagaaact 26280
   tctgtacgag tagaatgtga gcaaattatg tgttgaaatg gttcctctcc ctgcaggtct 26340
   ttcagctgaa acctggctta tctctcagaa gtactttcct tgcacagttt ctacttgtcc 26400
   ttcacagaaa agccttgaca ctaataaaat atatagaaga cgatacgtga gtaaaactcc 26460
   tacacggaag aaaaaccttt gtacattgtt tttttgtttt gtttcctttg tacattttct 26520
   atatcataat ttttgcgctt cttttttttt tttttttttt tttttttcca ttatttttag 26580
   gcagaaggga aaaaagccct ttaaatctct tcggaacctg aagatagacc ttgatttaac 26640
   agcagagggc gatcttaaca taataatggc tctggctgag aaaattaaac caggcctaca 26700
   ctcttttatc tttggaagac ctttctacac tagtgtgcaa gaacgagatg ttctaatgac 26760
   tttttaaatg tgtaacttaa taagcctatt ccatcacaat catgatcgct ggtaaagtag 26820
   ctcagtggtg tggggaaacg ttcccctgga tcatactcca gaattctgct ctcagcaatt 26880
   gcagttaagt aagttacact acagttctca caagagcctg tgaggggatg tcaggtgcat 26940
   cattacattg ggtgtctctt ttcctagatt tatgcttttg ggatacagac ctatgtttac 27000
   aatataataa atattattgc tatcttttaa agatataata ataggatgta aacttgacca 27060
   caactactgt ttttttgaaa tacatgattc atggtttaca tgtgtcaagg tgaaatctga 27120
   gttggctttt acagatagtt gactttctat cttttggcat tctttggtgt gtagaattac 27180
   tgtaatactt ctgcaatcaa ctgaaaacta gagcctttaa atgatttcaa ttccacagaa 27240
   agaaagtgag cttgaacata ggatgagctt tagaaagaaa attgatcaag cagatgttta 27300
   attggaattg attattagat cctactttgt ggatttagtc cctgggattc agtctgtaga 27360
   aatgtctaat agttctctat agtccttgtt cctggtgaac cacagttagg gtgttttgtt 27420
   tattttattg ttcttgctat tgttgatatt ctatgtagtt gagctctgta aaaggaaatt 27480
   gtattttatg ttttagtaat tgttgccaac tttttaaatt aattttcatt atttttgagc 27540
   caaattgaaa tgtgcacctc ctgtgccttt tttctcctta gaaaatctaa ttacttggaa 27600
   caagttcaga tttcactggt cagtcatttt catcttgttt tcttcttgct aagtcttacc 27660
   atgtacctgc tttggcaatc attgcaactc tgagattata aaatgcctta gagaatatac 27720
   taactaataa gatctttttt tcagaaacag aaaatagttc cttgagtact tccttcttgc 27780
   atttctgcct atgtttttga agttgttgct gtttgcctgc aataggctat aaggaatagc 27840
   aggagaaatt ttactgaagt gctgttttcc taggtgctac tttggcagag ctaagttatc 27900
   ttttgttttc ttaatgcgtt tggaccattt tgctggctat aaaataactg attaatataa 27960
   ttctaacaca atgttgacat tgtagttaca caaacacaaa taaatatttt atttaaaatt 28020
   ctggaagtaa tataaaaggg aaaatatatt tataagaaag ggataaaggt aatagagccc 28080
   ttctgccccc cacccaccaa atttacacaa caaaatgaca tgttcgaatg tgaaaggtca 28140
   taatagcttt cccatcatga atcagaaaga tgtggacagc ttgatgtttt agacaaccac 28200
   tgaactagat gactgttgta ctgtagctca gtcatttaaa aaatatataa atactacctt 28260
   gtagtgtccc atactgtgtt ttttacatgg tagattctta tttaagtgct aactggttat 28320
   tttctttggc tggtttattg tactgttata cagaatgtaa gttgtacagt gaaataagtt 28380
   attaaagcat gtgtaaacat tgttatatat cttttctcct aaatggagaa ttttgaataa 28440
   aatatatttg aaattttgcc tctttcagtt gttcattcag aaaaaaatac tatgatattt 28500
   gaagactgat cagcttctgt tcagctgaca gtcatgctgg atctaaactt tttttaaaat 28560
   taattttgtc ttttcaaaga aaaaatattt aaagaagctt tataatataa tcttatgtta 28620
   aaaaaacttt ctgcttaact ctctggattt cattttgatt tttcaaatta tatattaata 28680
   tttcaaatgt aaaatactat ttagataaat tgtttttaaa cattcttatt attataatat 28740
   taatataacc taaactgaag ttattcatcc caggtatcta atacatgtat ccaaagtaaa 28800
   aatccaagga atctgaacac tttcatctgc aaagctagga ataggtttga cattttcact 28860
   ccaagaaaaa gttttttttt gaaaatagaa tagttgggat gagaggtttc tttaaaagaa 28920
   gactaactga tcacattact atgattctca aagaagaaac caaaacttca tataatacta 28980
   taaagtaaat ataaaatagt tccttctata gtatatttct ataatgctac agtttaaaca 29040
   gatcactctt atataatact attttgattt tgatgtagaa ttgcacaaat tgatatttct 29100
   cctatgatct gcagggtata gcttaaagta acaaaaacag tcaaccacct ccatttaaca 29160
   cacagtaaca ctatgggact agttttatta cttccatttt acaaatgagg aaactaaagc 29220
   ttaaagatgt gtaatacacc gcccaaggtc acacagctgg taaaggtgga tttcatccca 29280
   gacagttaca gtcattgcca tgggcacagc tcctaactta gtaactccat gtaactggta 29340
   ctcagtgtag ctgaattgaa aggagagtaa ggaagcaggt tttacaggtc tacttgcact 29400
   attcagagcc cgagtgtgaa tccctgctgt gctgcttgga gaagttactt aacctatgca 29460
   aggttcattt tgtaaatatt ggaaatggag tgataatacg tacttcacca gaggatttaa 29520
   tgagacctta tacgatcctt agttcagtac ctgactagtg cttcataaat gctttttcat 29580
   ccaatctgac aatctccagc ttgtaattgg ggcatttaga acatttaata tgattattgg 29640
   catggtaggt taaagctgtc atcttgctgt tttctatttg ttctttttgt tttctcctta 29700
   cttttggatt tttttattct actatgtctt ttctattgtc ttattaacta tactctttga 29760
   tttattttag tggttgtttt agggttatac ctctttctaa tttaccagtt tataaccagt 29820
   ttatatacta cttgacatat agcttaagaa acttactgtt gttgtctttt tgctgttatg 29880
   gtcttaacgt ttttatttct acaaacatta taaactccac actttattgt tttttaattt 29940
   tacttataca gtcaattatc ttttaaagat atttaaatat aaacattcaa aacaccccaa 30000
   t 30001
<210> 3
   <211> 20
   <212> DNA
   <213> Artificial sequence
<220>
   <223> Primer
<400> 3
   gggtctagca agagcaggtg 20
<210> 4
   <211> 21
   <212> DNA
   <213> Artificial sequence
<220>
   <223> Primer
<400> 4
   gtcttggcaa cagctggaga t 21
<210> 5
   <211> 24
   <212> DNA
   <213> Artificial sequence
<220>
   <223> Probe
<400> 5
   tgatgtcgac tctttgccca ccgc 24
<210> 6
   <211> 18
   <212> DNA
   <213> Artificial sequence
<220>
   <223> Synthetic oligonucleotide
<400> 6
   gcccctagcg cgcgactc 18